# EUROPEAN PATENT SPECIFICATION

(11) **EP 1 745 764 B1**
(45) Date of publication and mention of the grant of the patent: **23.12.2015**
(21) Application number: 06123846.5
(22) Date of filing: 14.06.2002
(51) Int. Cl.: A61F 2/46

(54) **Impaction device for an implant**
Einschlagvorrichtung für ein Implantat
Appareil d'impaction d'un implant

(30) Priority: 14.06.2001 US 882591; 12.02.2002 US 75829
(43) Date of publication of application: 24.01.2007
(62) Divisional of application: 02742045.4
(73) Proprietor: Alexandria Research Technologies, Inc., Plymouth, MN 55447 (US)
(72) Inventor: Engh, Gerard, Remington, VA 22734 (US); Johnson, Wesley D., Eden Prairie, MN 55347 (US)
(74) Representative: Davies, Jonathan Mark

(56) References cited:
- DE-U- 29 609 632
- US-A- 5 352 230
- US-A- 5 474 560
- US-B1- 6 231 611

## Description

### Background of the Invention

A joint, such as the ankle, knee, hip or shoulder, generally consists of two or more relatively rigid bony structures that maintain a relationship with each other. Soft tissue structures spanning the bony structures ho Id the bony structures together and aid in defining the motion of one bony structure to the other. In the knee, for example, the bony structures are the tibia and the femur. Soft tissue such as ligaments, tendons, menisci, and capsule provide support to th e tibia and femur. A smooth and resilient surface consisting of articular cartilage covers the bony structures. The articular surfaces of the bony structures work in concert with the soft tissue structures to form a mechanism that defines the envelop of mo tion between the structures. Within a typical envelop of motion, the bony structures move in a predetermined pattern with respect to one another. When fully articulated, the motion defines a total envelop of motion between the bony structures. In the knee, the soft tissue structures spanning the joint tend to stabilize the knee in a transverse plane. This transverse stability enables the bony structures to slide and rotate on one another in an orderly fashion.

The articular surfaces are subject to a variety of diseases, accidents and the like that cause the surfaces to be damaged. A common disorder of joints is degenerative arthritis. Degenerative arthritis causes progressive pain, swelling, and stiffness of the joints. As the arthritic process develops, th e joint surfaces wear away, resulting in contractures of the surrounding soft tissues that provide stability to the joint. Changes in the articular surfaces resulting from arthritis decrease stability and increase the translation of the joint.

Treatment of the afflicted articular bone surfaces depends, among other things, upon the severity of the damage to the articular surface and the age and general physical robustness of the patient. The end result commonly necessitates joint replacement surgery wherein the articulating elements of the joint are replaced with artificial elements commonly consisting of a part made of metal articulating with a part made of ultra high molecular weight polyethylene (UHMWPE).

A relatively young patient with moderate to sever e degeneration of the knee joint is often treated with drug therapies. While drug therapies may temporarily provide relief of pain, progression of the disease, with resulting deformity and reduced function, ultimately necessitates surgery. Alternative treatments such as anti-inflammatory drugs, cortisone injections, and arthroscopic debridement similarly provide only temporary relief of symptoms.

In severe situations, the entire articular surface of a bone may be replaced with an artificial surface, as, for r example, when condyles at the distal end of the femur are largely replaced with a prosthetic device having polished metal condyles and the tibial plateau is replaced with a plastic bearing that may be supported by a metal component. Joint replacement sur gery has become a proven and efficacious method of alleviating pain and restoring function of the joint.

Current methods of preparing the rigid elements of a joint to receive components as in joint replacement surgery involve an extensive surgical exposure. The exposure must be sufficient to permit the introduction of guides that are placed on, in, or attach to the joint, along with cutting blocks to guide the use of saws, burrs and other milling devices, and other instruments for cutting or, removing cartilage and bone that subsequently is replaced with artificial surfaces. For knee joint replacement, the distal end of the femur may be sculpted to have flat anterior and posterior surfaces generally parallel to the length of the femur, a flat end surface normal to the anterior and posterior surfaces, and angled flat surfaces joining the above mentioned surfaces, all for the purpose of receiving a prosthetic device. For total hip replacement, the acetabular articular surface and subchondral bone is removed by spherical reamers, the femoral head is resected with an oscillating saw, and the proximal is shaped with broaches. A difficulty with total hip replacement is that the invasiveness of the procedure causes significant intraoperative blood loss and extensive rehabilitation because muscles and tendons must be released from the proximal femur to mobilize the femur and gain exposure of and access to the acetabular fossa.

A full joint replacement, using the example of the knee joint, also requires the proximal end of the tibia to be sculpted to receive a prosthesis having a generally upwardly facing bearing surface mimicking the normal tibial bearing surface and designed to articulate with the condylar surfaces of the femoral prosthesis. Typically, this surgery is performed with instruments or guides to orient cutting blocks, such that the preparation of the bone is in concordance with the correct alignment of the limb and the parts are correctly oriented in both coronal and sagittal positions. The guides are placed on exposed bones and generally reference anatomical points on that bone to establish a resection plane. For instance, with total knee replacement, arthroplasty guides are used by referencing, for example, the intramedullary cavity and the epicondylar an d posterior condylar axes.

Knee joint prosthesis of the type referred to above are well known, and are described, for example, in et. U.S. patents 5,171,244, 5,171,276 and 5,336,266, Brown, U.S. 547, Burstein et al., U.S. patent 4,298,992, and Insall et al., U.S. patent 6,068,658.

Substantial effort has been made to provide appropriate degrees of curvature to the condyles in knee joint replacement. For example, the earlier mentioned U.S. patents 5,171,276, 4,298,992 and 6,068,658 show that the radius of curvature in the anterior-posterior direction of the condyle of a femoral prosthesis may be somewhat greater near the anterior portion of the condyle than near the posterior portion. Kester et al., U.S. Patent 5,824,100 teaches that a portion of this curvature of the condyle may be formed about a constant radius having its origin along a line between the lateral and medial collateral ligament attachment points on the femur.

Historically, a variety of modular prosthetic joint implants have been develo ped. The following descriptions of modular implants relate specifically to the knee. Early designs for knee implants, called polycentric knee implants, were developed with separate components for the medial and lateral compartments. Additionally, modular fixed-bearing knee implants having a polyethylene insert that is held relatively rigidly in place have been developed. Alternately, there are mobile bearing knee implants wherein the polyethylene bearing is designed to slide or move with minimal or no constraint on a tibial baseplate. Furthermore, both meniscal bearing and fixed bearing knee implants have been developed including either separate polyethylene bearings or a single polyethylene bearing that resides on a metallic tibial baseplate. While implant systems have been developed with fixed bearing elements or mobile bearing elements on the medial and lateral sides of the tibiofemoral joint, systems have not been developed having a combination of a fixed bearing on one side and a mobile bearing on the other side of the tibiofemoral joint.

Mobile bearing tibial implants may be configured to be more congruent with the femoral side of a knee arthroplasty, yielding lower contact stress. The resultant lower contact stress reduces the possibility of damage so metimes encountered with some fixed bearing designs wherein the yield strength of the bearing material is exceeded. In general, fixed bearing implant designs are less difficult to properly align and balance than mobile bearing designs. Mobile bearing desig ns are frequently desirable to reduce contact stress and the resulting wear of the bearing surface. However, with mobile bearing designs, there is the possibility of the bearing becoming dislodged from the implant. Additionally, mobile bearing knee designs are more surgically demanding to implant then fixed bearing designs.

The combination of a fixed bearing insert for the medial compartment and a mobile bearing insert for the lateral compartment is particularly attractive because the lateral femoral condyle rolls backward on the lateral tibial plateau as much as 10 to 20 millimeters whereas the medial condyle moves only a few millimeters. A mobile bearing insert is able to accommodate the rollback of the lateral condyle but would not be necessary for the medial condyle.

Two primary difficulties exist with current joint replacement surgeries. These relate to the invasiveness of the procedure and achieving proper alignment -of the bony structures and the prostheses thereupon. Such difficulties are present in all total joint replacements, including ankle, knee, hip and shoulder. Total knee and total hip are described as general examples of the difficulties in current joint replacement surgery.

Alignment. A difficulty with implanting both modular and non -modular knee implants having either separate femoral and/or tibial components has been achieving a correct relationship between the components. Surgical instruments available to date have not provided trouble free use in implanting multi -part implants wherein th e femur and tibia are prepared for precise component -to-component orientation. While alignment guides aid in accurate orientation of the components relative to the axis of the long bones to achieve a restoration of a correct alignment (usually 4 - 7 degrees valgus), they provide limited positioning or guidance relevant to correct component -to-component alignment and/or ligament tension to restore alignment.

It is preferable to orient implants normal to the resultant forces through the joint to subject bearing surfaces to compressive rather than shear forces. Moreover, the components of the implant are preferably oriented one to the other to minimize wear. Complications may result if the implant is not correctly oriented with respect to the supporting bone. I f the implant is not placed normal to the mechanical axis, a shearing force results between the implant and bone that may lead to implant loosening.

In a properly aligned knee, the mechanical axis of the leg (a straight line drawn from the center of the hip joint to the center of the ankle) passes slightly medial to the center of the knee. This alignment is generally called the gross alignment of the leg. The alignment of the implants impacts the gross alignment of the leg. If the implants are malaligned, the resulting mechanical axis may be shifted medially or laterally, resulting in an imbalance in the loads carried by the medial or lateral. This imbalance, if severe, may lead to early failure of the implant.

In addition, the orientation of the components to each other, for example the orientation of the femoral component to a second and or third femoral component, orientation of a tibial component to a separate second tibial component and orientation of a femoral component to its corresponding tibial com ponent, with and implants has largely not been addressed. This may account for the high failure rates of early bicondylar designs and as well as for the higher failure rate of implants relative to total knee implants as demonstrated in some clinical studies. When considering bicondylar and designs, alignment of each part relative to the other parts is critical to avoid accelerated wear with a mal-articulation of the components.

Although various prosthetic devices have been successfully used with patients, the configuration and position of the articulating surfaces of the prosthesis, for example the condyles in a knee joint, are predetermined based upon the prosthesis that is selected. While efforts are made to tailor the prosthesis to the needs of each patient by suitable prosthesis choice and size, this in fact is problematical inasmuch as the joint physiology of patients can vary substantially from one patient to another.

Invasiveness. In order to appropriately sculpt the articulating surface of a bone, it is often necessary to surgically expose the joint. In the case of the femur in traditional knee joint replacement, the patellar tendon of the knee joint is surgically exposed and is moved to one side of the joint to enable a substantially full anterior ac cess to the joint. Surgical exposure is necessary to accommodate the bulk and geometry of the components as well as the instruments for bone preparation. Such surgical exposure increases bleeding, pain, and muscle inhibition; all of which contribute to a longer hospitalization before the patient can be safely discharged to home or an intermediate care facility.

Desirably, in the case of knee replacement surgery, neither the collateral ligaments nor the cruciate ligaments are disturbed, although it is often necessary to remove or release cruciate ligaments in the event a substantial joint replacement is to be performed. Collateral ligaments can be partially taken down or released to provide appropriate tension adjustment to the patient's knee in concert with joint replacement surgery. In most instances, such releases can be accomplished through smaller incisions than the standard midline or medial parapatellar incisions historically used for knee arthroplasty.

Arthroscopic surgery is available, and beneficial, for removing and repairing damaged intraarticular tissues. Although arthroscopic procedures are far less invasive and are often successful for minor surgical repairs, (as when an articular surface is to be smoothed, for example, or cartilage is to be re paired), such procedures generally are not appropriate for substantial joint replacement. They are generally inadequate for replacing joint surfaces with artificial implants.

Conventional surgical procedures including unicompartmental and total joint replacement historically required extensive surgical exposure and prolonged hospital stays and rehabilitation. More recently, unicondylar knee joint replacement procedures have been performed through smaller incisions that do not necessitate dislocation of the patella. The reduction in pain and more rapid recovery of knee function has reduced the length of hospital stay and the need for strong narcotic medications. It is desirable to realize such benefits for patients with bicompartmental and tricompartmental k nee arthroplasty.

As in the knee, conventional total hip arthroplasty is indicated for painful arthritis of the hip joint. The procedure involves exposing the hip joint through a large incision to provide the surgeon full visualization of the hip joint an d the acetabular region and to provide access for surgical power instruments. In order to appropriately prepare the bony structures of the hip joint, the major muscles spanning the joint are commonly disrupted to gain adequate exposure of the joint. Steps of the procedure include removing the femoral head followed by reaming and broaching the proximal femoral canal to prepare a bony surface to support a hip stem. The stem is implanted and may be cemented in place, or press fit for bony ingrowth. The acetabulum is typically prepared using a hemispherical reamer to remove cartilage down to bleeding bone. Once the acetabulum is prepared, an acetabular component is implanted, either by cementing in place or press fitting for bony ingrowth. The surgical exposure , which may be between six and twelve inches in length, may result in extensive trauma to the soft tissues surrounding the hip joint along with the release of muscles that insert into the proximal femur.

The prepared bony surfaces are technically referred to as the acetabular fossa, femoral canal and metaphyseal region of the femur. Prior to placing final implants into the prepared spaces, a femoral trial, which may be a broach in some systems, is placed in the proximal femur along with a trial femoral head and neck, and an trial is placed into the acetabulum to facilitate trial range of motion and evaluation of hip stability prior to placement of the final total hip implants.

A system to enable minimally invasive total hip arthroplasty that will minimize soft tissue trauma and accelerate postoperative rehabilitation is needed. Further, because minimally invasive techniques inherently limit observation of the surgical site, compromising visualization of the prepared bony surfaces, a device is also needed fo r inspection of the prepared bony surfaces. During a surgical procedure, bone debris and blood will gather in the surgical site and require removal from time to time to visualize the acetabulum. After preparation of the acetabulum, an acetabular component is implanted. A variety of acetabular components such as cemented UHMWPE cups, cemented or press fit metal shells with UHMWPE, metal, or ceramic bearing liners are presently used. Typically, placement of a press fit shell requires an impaction force to fully seat the implant into support bone. However, the size and location of the minimally invasive incision may not be optimal for proper orientation and application of force to adequately seat and stabilize an acetabular implant. Thus, an impaction device is needed that allows for impaction of the acetabular component with the hip reduced or articulated for use with a minimally invasive exposure for total hip arthroplasty. It may also be desirable to use a surgical navigation system to prepare the joint surfaces and position the implants.

For patients who require articular surface replacement, including patients whose joints are not so damaged or diseased as to require whole joint replacement, it is desirable to provide surgical methods and apparatuses that may be employed to gain surgical access to articulating joint surfaces, to appropriately prepare the bony structures, to provide artificial, e. g., metal or plastic, articular bearing surfaces, and to close the surgical site, all without substantial damage or trauma to associated muscles, ligaments or tendons. To attain this goal, a system and method is needed to enable articulating surfaces of the joints to be appropriately sculpted using minimally invasive apparatuses and procedures.

US 6231611 discloses the features of the preamble of the appended independent claim.

US 5 352 230 discloses an impaction device for imparting force to seat an implant in the femur, including an attachment portion for attaching it via the implant to the femur.

### Summary of the Invention

The invention provides an apparatus for imparting force between first and second adjacent bones that seat an implant in the first bone as defined in the appended independent claim. Preferred and advantageous features of the invention are set out In various dependent subclaims.

The present disclosure provides a system and method for total Joint replacement that involves minimally invasive surgical procedures including an implant system that restores individual patient joint kinematics. The instruments and implants disclosed accomplish accurate bone and soft tissue preparation, implant orientation and implant fixation through limited surgical exposure.

Thus, the present disclosure provides a method of appropriately sculpting the articular surface of a first b one that normally articulates with a second bone and implanting a prosthetic device. The method involves attaching a bone sculpting tool directly or indirectly to the second bone with the tool in bone sculpting engagement with the articular surface of the first bone, and then sculpting the articular surface of the first bone with the joint reduced and if indicated moving one bone with respect to the other. Optionally, the bone sculpting tool may be attached to a mount that is attached directly or indirectly to the second bone. In some situations, it may be desirable to distract the first bone from the second bone during surgery. The bone sculpting tool may also be attached to a bone mount that is directly or indirectly attached to or integral with a stem, trial, reamer or broach implanted in the medullary canal of a bone.

For knee joint replacement, the Implant system is comprised of implants and Instrumentation that provide intraoperative surgical options for articular constraint and facilitate proper alignment and orientation of the knee to restore kinematics as defined by the individual patient anatomy. To do so, the implants provide a surgeon intraoperative options to reconstruct various degrees of joint stability via selection of fixed or mobile bearing components for each compartment of the knee (medial joint, lateral libiofemoral joint and patellofemoral joint). The range of implants may cover only one compartment or each compartment of the knee and may include combinations of fixed and mobile bearing configurations.

In traditional total knee replacements, the femoral component is generally a unitary piece and the tibial component is a unitary piece. In the disclosed knee replacement, the femoral side may be resurfaced by two or three components and the tibial s ide may be resurfaced by two components or a unitary piece. The components may be aligned and or joined one to another within the confines of the joint. Optionally, the components of the femoral side may be comprised of a plurality of flexible segments.

Proper alignment and positioning of the implant components is facilitated by instrumentation that utilizes the soft tissue structures of the knee to guide bony resections for patient-specific alignment and orientation of the implants. Alignment of the implants with respect to the supporting bones relates to restoring or improving the anatomical alignment of the supporting bones. Orientation of the implants with respect to the supporting bones is significant in two ways. First, orientation of the implant construct with respect to the supporting bones is such that the forces transferred through the implant are generally normal or perpendicular to the bony support surfaces. Second, orientation of the implant components with respect to one another is such that each modular component restoring the femoral articular surfaces is properly aligned with the other modular components on the femoral side to ensure proper tracking of the implant construct. Likewise, each modular component restoring the tibial articular surfaces is properly aligned with other modular components on the tibial side to ensure proper tracking of the implant construct. The surgical instrumentation prepares the articular surfaces of a synovial joint from a single point of reference to allow the introduction of separate components for the medial and lateral tibiofemoral compartments, and the patellofemoral compartments with precise orientation. Thus, the instrumentation provides bony resections in accordance with such alignment and orientation requirements. The positioning is important for proper restoration of anatomic alignment of the knee joint and for proper orientation of the components to one 'another.

With respect to forming or sculpting articular surfaces of a joint, the method of the current disclosure enables the articular bone surfaces to be sculpted according to the individual physiology of each patient to restore as much as possible the natural junction of the joint. In this method, a bone sculpting tool is attached to one of the bones of a joint, and the tool sculpts the articular surface of the other bone as the joint is articulated.

Thus, the present disclosure provides a method of appropriately sculpting the articular surface of a first bone that normally articulates with a second bone. The method involves providing an apparatus comprising a bone sculpting tool attached to a bone mount, attaching the mount rigidly to the second bone with the tool in bone sculpting engagement with the articular surface of the first bone, and then sculpting the articular surface by articulating one of the bones with respect to the other.

In some situations, it may be desirable to distract the first bone from the second bone, either or during surgery. Thus, a distractor may be provided wi th the apparatus. In knee joint replacement, a distraction force provided between the femur and the tibia during the sculpting procedure accounts for material that has worn away from the articular surfaces. Use of a distraction force generally re -establishes normal alignment of the joint. Ligament releases may be carried out to restore alignment either prior to preparing the joint surfaces or following the preparation of the joint surfaces with bone sculpting instruments. Additionally, a distractor may be u sed preoperatively to assess the range of motion of the joint and patient kinematics.

The disclosure provides an apparatus for sculpting the articular surface of a first bone that normally articulates in a predetermined manner with a second bone. The apparatus comprises a bone sculpting tool, a mount attachable rigidly to the second bone, and an adjustable attachment attaching the sculpting tool to the mount and enabling the position and orientation of the tool to be adjusted into bon e-sculpting proximity to the articular surface so that the articular surface is sculpted as the second bone is articulated with respect to the first bone. Alternately, a plurality of bone sculpting tools may be used where the tools are positioned either o n individual mounts or on a single mount to support the plurality of tools.

The disclosure also provides implants for replacing the surfaces of the joint between the first bone and the second bone. The implants are specifically designed to fit through mini mally invasive incisions and incorporate any and all combinations of fixed and mobile bearing inserts or parts. Since the surgical procedure preferably is performed through minimally invasive incisions the implants are designed to fit through such incision s and be either oriented or joined within the joint.

In the case of knee replacement surgery, the implants include a second bone baseplate and a first bone implant. The second bone baseplate may be either one piece to cover most of the prepared surface of the second bone as relates to the joint, or separate baseplates as have been used with mobile and fixed bearing prosthetic components. In addition, the second bone baseplate may accommodate separate fixed and mobile bearing inserts. The first bone implant is comprised of a plurality of components to replace the bearing surface of the first bone. Optionally, a portion of the first bone implant may be configured of a plurality of flexible segments bonded in place. Such a configuration permits the articulation of the second bone to the first bone to mould the flexible segments in appropriate position.

Thus, the disclosure provides a method of appropriately replacing the articular surface of a first bone that normally articulates with a second bone. The method involves providing an apparatus comprising a bone sculpting tool attached to a bone mount, attaching the mount rigidly to the second bone with the tool in bone sculpting engagement with the articular surface of the first bone, and then sculpting the articular surface by articulating one of the bones with respect to the other. Further, the articular surfaces are resurfaced with appropriate minimally invasive implants wherein the implants are joined within the confines of the joint ca vity. In one replacement, a plurality of flexible segments are provided to resurface a portion of the first bone. The flexible segments are set in an adhesive along the resected surface of the first bone.

Specifically, for example in knee joint replacement, the system may be used for replacing the surfaces of a femur and a tibia. Thus, a femoral implant having a plurality of components and a tibial baseplate are provided. The tibial baseplate may have a fixed bearing attachment as well as a mobile bearing attachment.

When applied to total hip joint replacement, the system may be used for replacing the surfaces of a femur and through a minimal incision and with minimal disruption of structures about the hip. A typical incision for a minimally invasive total hip procedure is between two and four inches in length. It is noted that there may be some variation in incision length due to patient physiology, surgeon preferences, and/or other factors; the stated range is illustrative, not limiting. In addition to a small incision, care is taken to approach the joint capsule by separating tissues between muscle groups, rather than sectioning specific muscles.

In total hip replacement, an acetabular component, such as a press fit shell, is implanted following preparation of the acetabulum. An impaction device is provided that allows for impaction of the component with the hip reduced or articulated in order to fully seat a press fit acetabular component into support bone of the A surgical navigation system for pos itioning the acetabular component may be used with the impaction device.

In the minimally invasive procedure, the hip is accessed through an incision adequate to expose the trochanteric fossa and allow resection of the femoral neck and removal of the femoral head and neck segment. The femoral canal is accessed through the trochanteric fossa and trochanteric region. Reamers, rasps and other devices as are known to those skilled in the art are used to prepare the proximal femur to receive a femoral implant by a sequence of reaming and broaching steps. Once prepared, the intramedullary canal and retained area of the femoral neck and trochanteric region are used to support the MIAR (Minimally Invasive Reamer) system to prepare the acetabulum.

### Brief Description of the Drawings

Figure 1 shows a plan view of knee joint.
Figure 2 shows a traditional midline incision for accessing the knee joint during total knee replacement surgery.
Figure 3 shows an incision for accessing the knee joint during total knee replacement surgery that may be used with the disclosed method and apparatus.
Figure 4 shows alternate incisions for accessing the knee joint during total knee replacement surgery that may be used with the method and apparatus.
Figure 5 illustrates a cross -sectional view of the cavity created in the tibial plateau in accordance with one method.
Figure 6 shows a plan view of an instrument for creating a resection in the tibial plateau.
Figure 7 shows an end view of the instrument of Figure 6.
Figure 8 shows a plan view of a second instrument for creating a resection in the tibial plateau.
Figure 9 shows an end view of the instrument of Figure 8.
Figure 10 shows a plan view of tibial resections.
Figure 11 shows a top view of the resections shown in Figure 10.
Figure 12 shows a cross -sectional view of one of the resections shown in Figure 10.
Figure 13 shows a plan view of a cutting element attached to a tibial resection.
Figure 14 shows a side view of a configuration of the cutting element of Figure 13 connected to a motor.
Figures 15 and 16 show alternate views of a cutting element driven by a hydraulic motor.
Figures 17 and 18 show alternate views of a cutting element driven by an alternate hydraulic motor.
Figure 19 shows an end view of a cutting element.
Figure 20 shows a side view of the cutting element of Figure 19.
Figure 21 shows an end view of a second cutting element.
Figure 22 shows a side view of the cutting element of Figure 21.
Figure 23 shows an end view of a third cutting element.
Figure 24 shows a side view of the cutting element of Figure 23.
Figure 25 shows a side view of a fourth cutting element.
Figure 26 shows an end view of the cutting element of Figure 25.
Figure 27 shows a cross -sectional view of the cutting element of Figure 25.
Figure 28 shows an end view of a fifth cutting element.
Figure 29 shows a cross -sectional view of the cutting element of Figure 28 with a single cutting element.
Figure 30 shows a cross -sectional view of the cutting element of Figure 28 with multiple cutting elements.
Figure 31 illustrates the kinematics of the articulation of the knee joint with a cutting element.
Figure 32 shows a plan view of two cutting element linked by a hinge mechanism.
Figure 33 shows a plan view of an alternate state of the two cutting element linked by a hinge mechanism of Figure 32.
Figure 34 shows a plan view of a different pair of cutting elements linked by a hinge mechanism
Figure 35 shows a plan view of the cutting elements of Figure 34.
Figure 36 shows a plan view of two cutting elements linked by a hinge mechanism deployed in the knee joint.
Figure 37 shows a sectional view of a cutting element and distractor.
Figure 38 shows a top view of the cutting element of Figure 37.
Figure 39 shows a plan view of distractors deployed in the knee joint.
Figure 40 shows a plan view of femoral resections.
Figure 41 shows a plan view of alternate femoral resections containing femoral implants.
Figure 42 shows a plan view of yet another pair of femoral resections with a containing femoral implants.
Figure 43 shows plan views of alternate tibial baseplates.
Figure 44 shows a plan view of femoral implants for resurfacing the femoral resections of Figure 38k.
Figure 45 shows a plan view of femoral implants for resurfacing the femoral resections of Figure 41.
Figure 46 shows a plan view of a femoral implant.

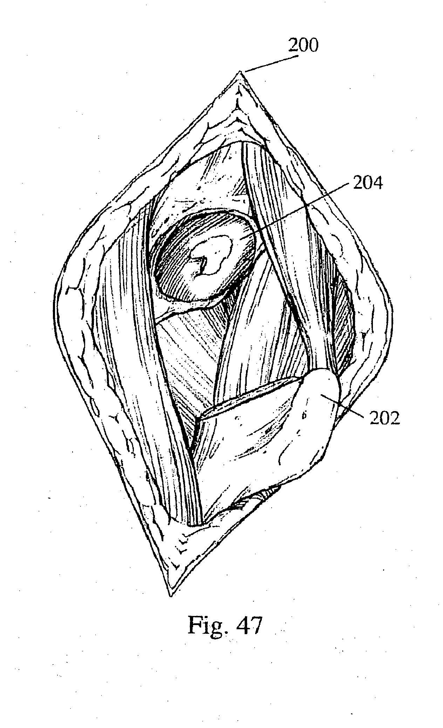

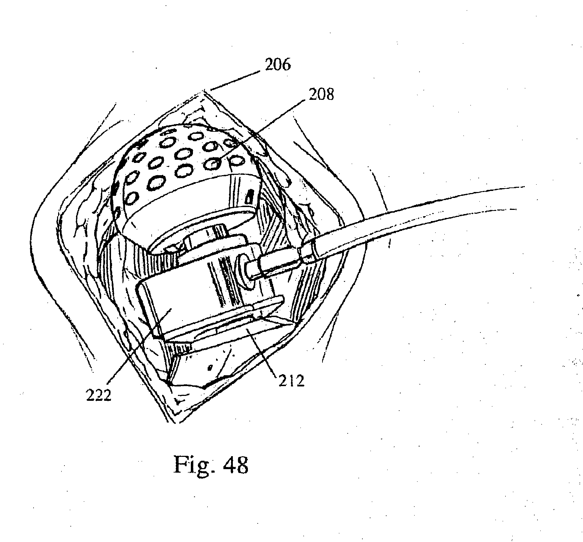

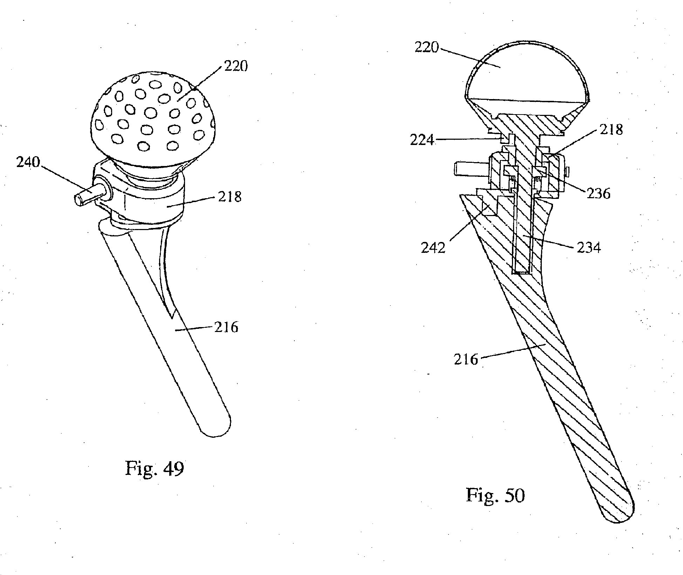

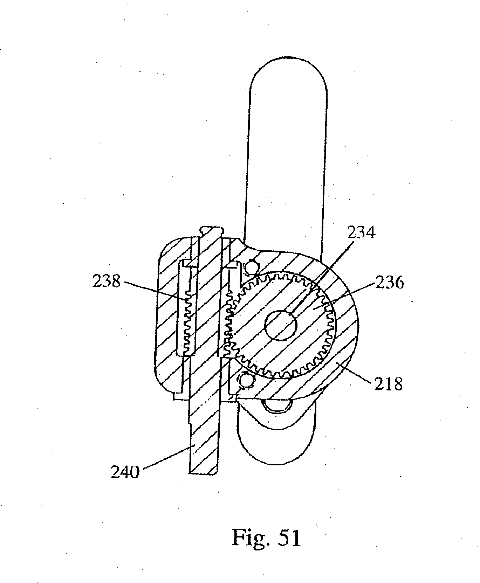

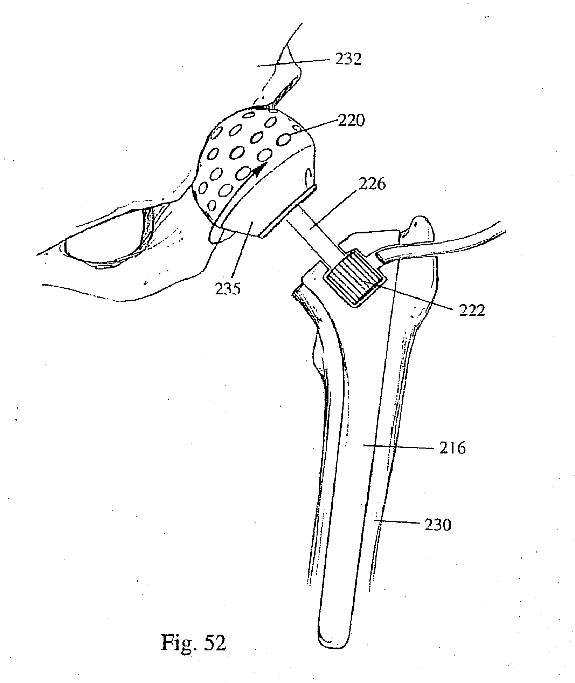

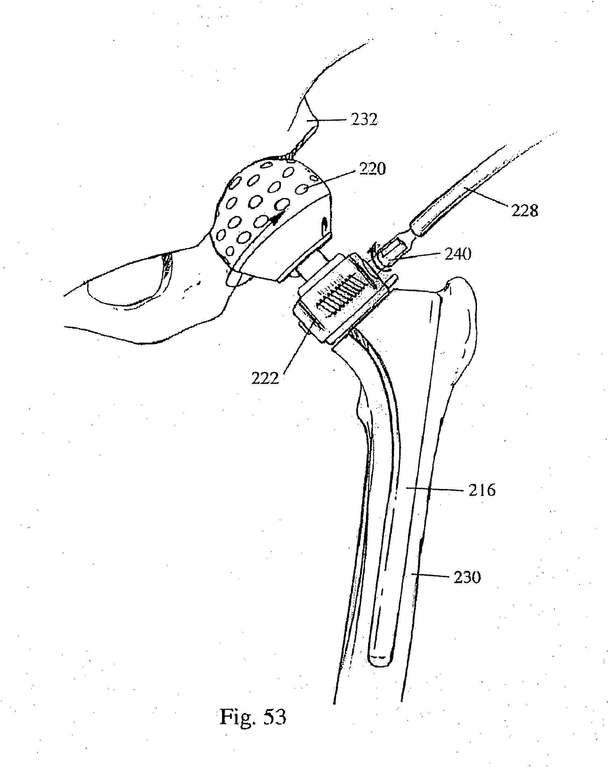

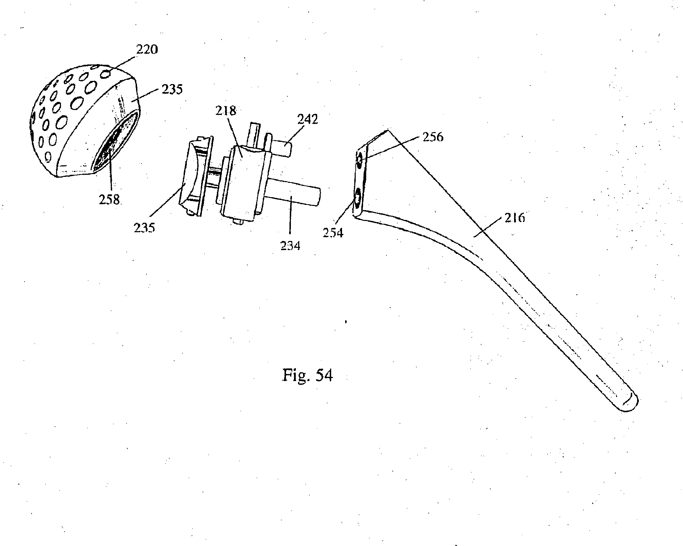

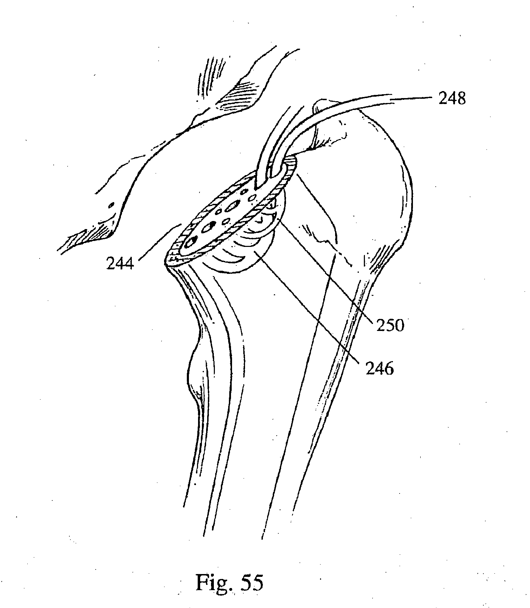

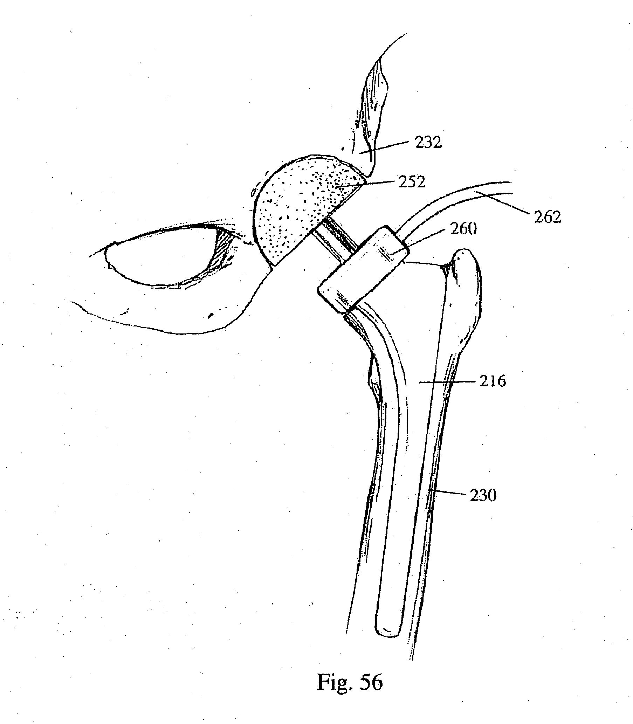

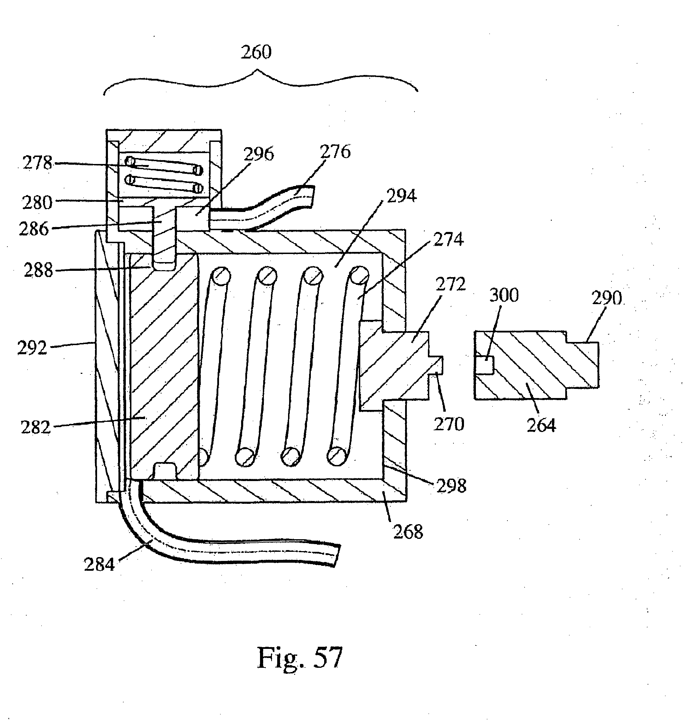

Figure 47 is an illustration of hip anatomy and conventional exposure for total hip replacement Figure 48 is an illustration of exposure for minimally invasive total hip replacement with reamer; Figure 49 is an illustration of a minimally invasive acetabular reamer; Figure 50 is a cross sectional view of a minimally invasive acetabular reamer in a sagittal plane; Figure 51 is a cross sectional view of a mi nimally invasive acetabular reamer cross in a transverse plane; Figure 52 is an illustration of a minimally invasive acetabular reamer with an integral hydraulic drive; Figure 53 is an illustration of a minimally invasive acetabular reamer with a worm gear drive mechanism; Figure 54 is an expanded view of a minimally in vasive acetabular reamer; Figure 55 is an illustration of illumination, visualization, irrigation and suction of an operative site; Figure 56 is an illustration of a minimally invasive impaction system in accordance with the present invention; and Figure 57 is a detailed depiction of a minimally invasive impactor in accordance with one embodiment of the present invention.

### Detailed Description

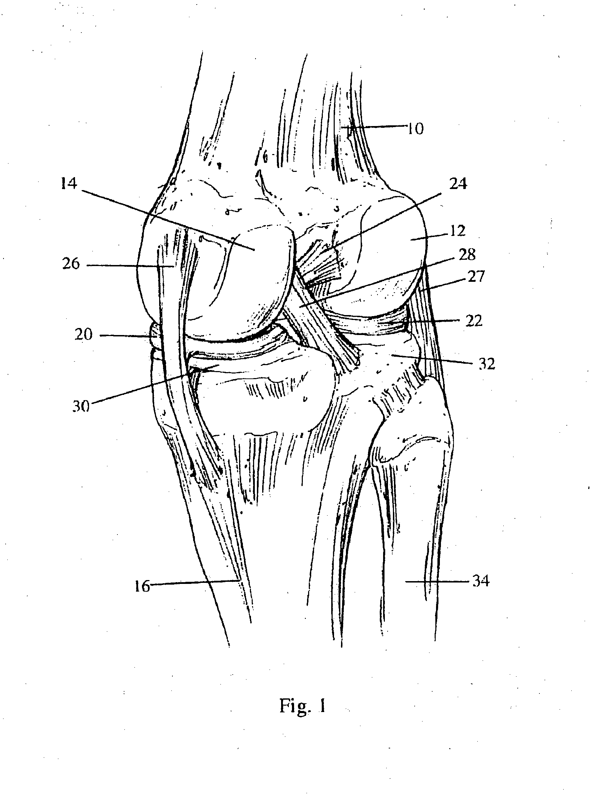

Knee Joint Anatomy and Surgical Approaches . Figure 1 illustrates the general anatomy of the knee joint. The femur 10 has the lateral femoral condyle 12 and the medial femoral condyle 14 on its knee joint articul ating surface. The tibia 16 has the lateral meniscus 22 (generally opposite the lateral femoral condyle 12) and the medial meniscus 20 (generally opposite the medial femoral condyle 14) on its knee -joint articulating surface. The ligaments include the ante rior cruciate ligament 24, the posterior cruciate ligament 28, the medial collateral ligament 26 and the lateral collateral ligament 27. The medial tibial condyle 30 and the lateral tibial condyle 32 support the menisci 20 and 22, which in turn support the femur 10. Additionally, the fibula 34 engages the tibia 16.

Typically, a total knee joint replacement involves replacing the articular surfaces of the lateral femoral condyle 12, the medial femoral condyle 14, the medial tibial condyle 30 and the latera I tibial condyle 32. The lateral meniscus 22, and the medial meniscus 20 are removed. Desirably, neither the collateral ligaments 26 and 27 nor the cruciate ligaments 24 and 28 are disturbed. However, the collateral ligaments 26 and 27 may be partially taken down to provide appropriate tension adjustments to the patient's knee after joint replacement has been completed.

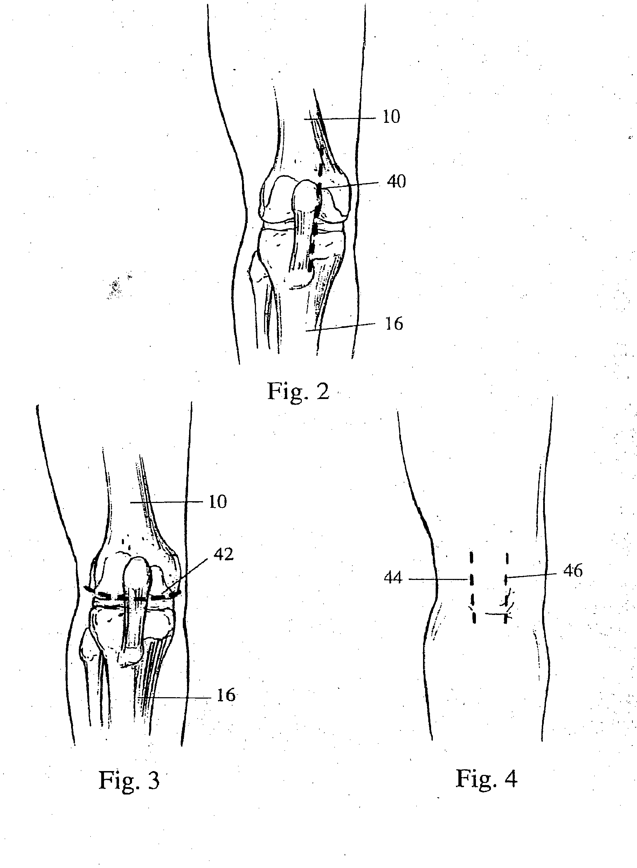

Figure 2 illustrates the conventional midline incision 40 for a total knee replacement surgery. The incision 40 extends vertically substantially above and below the articulating surface between the femur and the tibia. Typically, the incision is roughly 8 to 15 centimeters in length. The incision 40 must be large enough to expose the entire knee joint articular surfaces with the patella subl uxed or dislocated. Additionally, the incision must accommodate insertion of components that fully cover the end of the femur, the top of the tibia and the undersurface of the patella. The maximum number of components implanted would include femoral and ti bial components for the lateral tibiofemoral compartment, femoral and tibial components for the medial tibiofemoral compartment and femoral and patellar components for the patellofemoral joint. Alternatively, the lateral femoral condyle and the patellar gr oove may be covered by a common implant.

As seen in Figure 3, a transverse incision 42 extending horizontally along the knee joint is one option for the procedure. The incision 42 may be vertically opened to expose the joint surfaces of the compartment and the lateral tibiofemoral compartment without dislocating the patella. This maintains the patella in contact with the femur during the procedure. The components of the instrumentation as well as the implant are sized for minimal invasiveness and, therefore, may be accommodated by the small incision. The reduced trauma resulting from a smaller incision generally results in faster and better rehabilitation, which in turn generally increases the efficacy of the knee implant.

Figure 4 depicts an alternate incision format. Two parallel vertically extending incisions 44 and 46 may be formed on either side of the patella. These incisions 44 and 46 are relatively short and the invasiveness is similar to that of the horizontal incision in Figure 3. Each incision 44 and 46 is separately extended through the joint capsule to expose the medial and lateral tibiofemoral compartments without dislocating the patella.

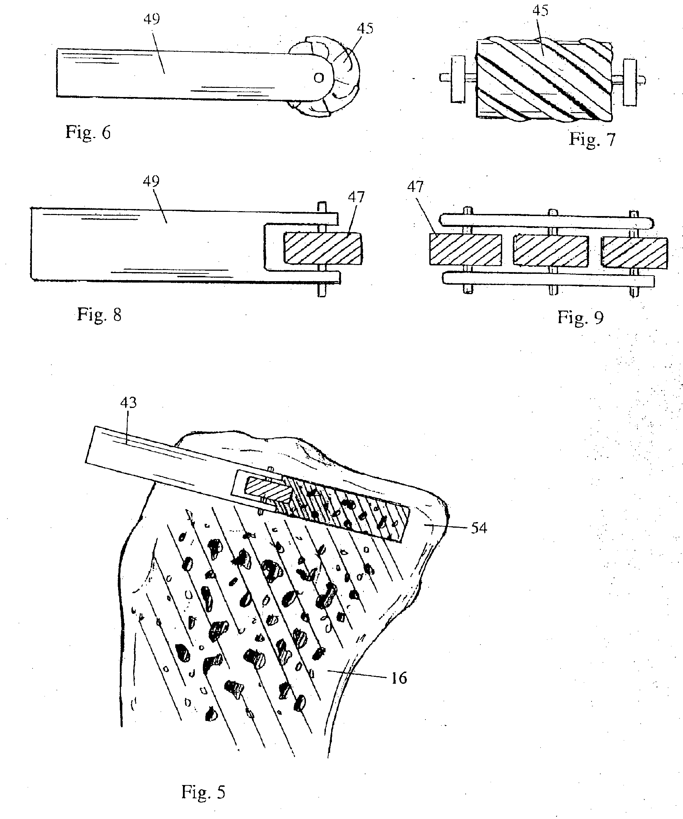

Instrumentation. The instrumentation, as related to total knee replacement, generally calls for resecting the tibia at the lateral tibial plateau and the medial tibial plateau. Optionally the instrumentation can be used to resect the distal femur thereby creating an extension space to accommodate bone sculpting instrumentation. This resection may be done by methods known by those skilled in the art, using a resection guide, saw, etc. Alternately, as shown in Figure 5, a milling burr 43 may be advanced directly into the tibia 1 6. The milling burr 43 should stop at or short of the posterior cortical wall 54. Figure 5 shows a cross sectional view through the cavity created in the tibial plateau by the milling burrs 47 of Figures 6 and 7.

As seen in Figures 6 and 7, the cutting de vice may be a single milling burr 45 affixed at the forward end of a guide element 49. The milling burr 45 of Figures 6 and 7 has its axel in a medial to lateral direction when preparing the tibial plateau.

The radius of the milling burr leaves a corresponding radius between the floor and posterior wall of the cavity created.

Alternately, as seen in Figures 8 and 9, the cutting device may comprise a plurality of milling burrs 47. The milling burrs 47 of Figures 8 and 9 prepare a corner between the floor a nd posterior wall of the cavity created in the tibial plateau. The corner thus prepared may distribute stress uniformly into the supporting bone. The milling burrs 47 create a radius equivalent to the radius of the burr between the sidewalls of the cavity and the posterior wall. Such a radius is easily accommodated by the tibial implant design. While Figures 8 and 9 depict a cutting device having a plurality of milling burrs, the cutting device may be configured with one milling burr.

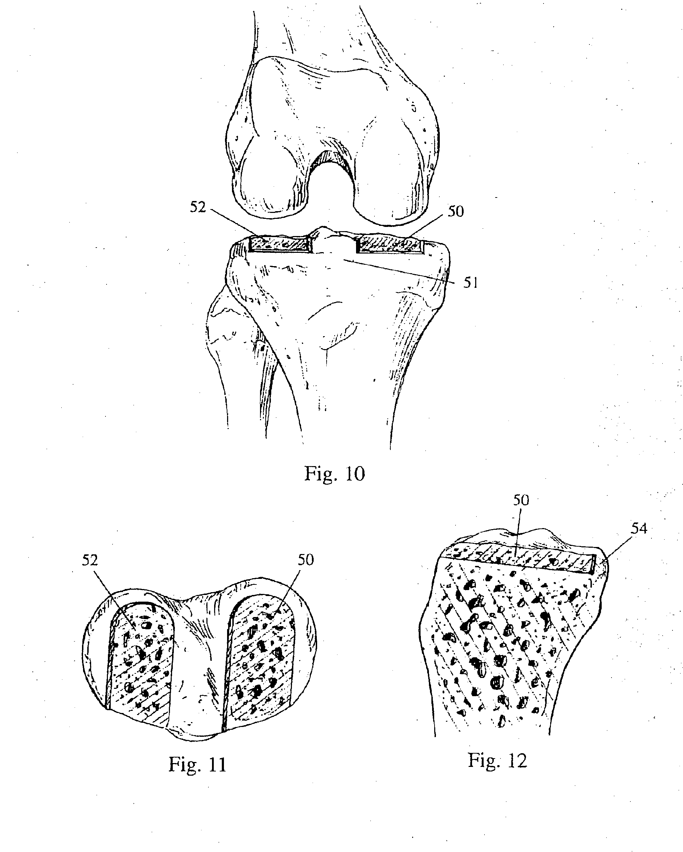

Figure 10 shows an an terior view of the bone resections 50 and 52 that are made in the tibial plateau, generally 51. The floor of the medial resection 50 and the floor of the lateral resection 52 are preferably parallel and co -planar to ensure proper alignment and orientation of the medial and lateral tibial components. The external tools used to guide the tibial cutter may provide relative alignment between the medial and lateral resections. Alternately, the medial and lateral cavities in the tibial plateau may be prepared sim ultaneously by having two guide elements 49 linked together by a hinge that restrains the medial and lateral milling burrs 47 in a common plane. The external tools may further provide a positive reference to the posterior aspect of the tibial plateau to en sure that the resections do not penetrate the posterior cortical wall. In Figure 10, the bone resections are shown to have a generally rectangular cross - section. However, any cross -section to which a bone sculpting tool may be mounted may be used. For example, an arcuate cross-section is acceptable.

Figure 11 shows a top-view of bone resections 50 and 52 in the tibia 16. A cross-sectional view of the tibia 16 with a cavity machined into the plateau is depicted in Figure As seen in Figure 12, the bone resection 50 should stop at or short of the posterior cortical wall 54.

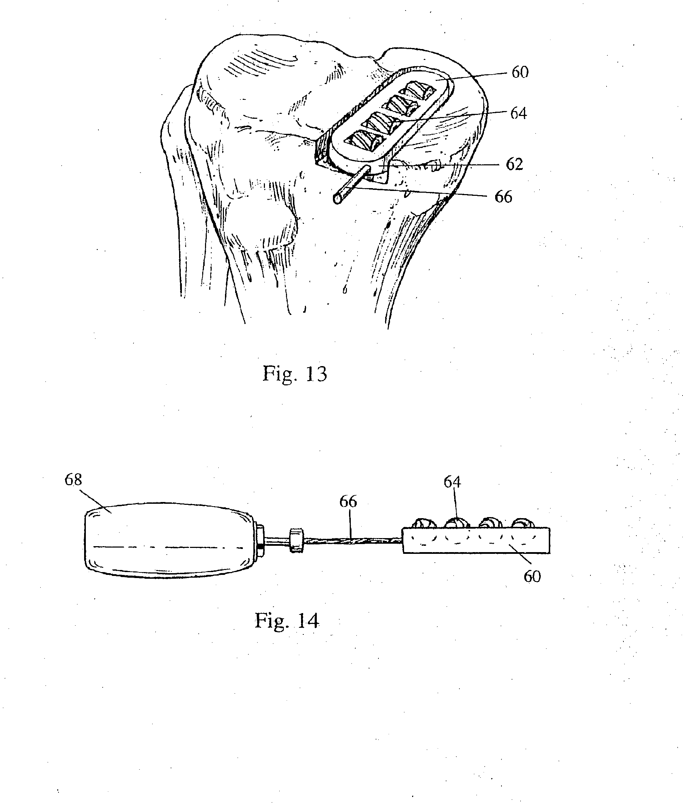

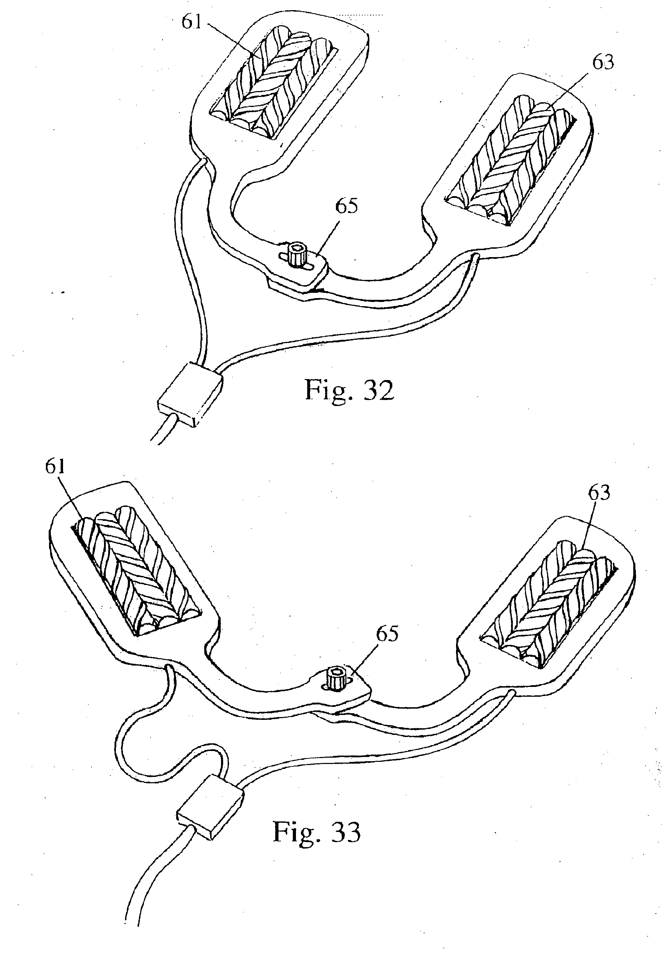

As seen in Figure 13, upon resection of the tibia, a bone sculpting tool, for example, a femoral cutter, generally 60, is placed in a mount and rigidly attached to the cavity created in th e tibia. Rigid attachment generally means providing sufficient stability to prevent relative motion between the mount and the tibia during articulation. Such stability may be provided through mere placement of the device in the tibial resection. The femora I cutter is designed to reference the tibial resections 50 and 52 when making the femoral resections. In one cutter, illustrated in Figure 13 the mount is a cradle 62 and is set in the resected tibia. Cutting elements 64 are mounted in the cradle 62 and a flexible shaft 66 connects the cutting element to the motor 68 of Figure 14. The device fits into the resections 50 and 52 in the medial and lateral tibial plateaus. Thus, a cutting element is rigidly held against the femoral condyle and the guide surface of the device sets the depth of resection. Optionally, a second cutting element may be placed in the opposite tibial resection. Thus, for example, two cutting elements may be placed in the prepared tibial plateau, one in the medial cavity and one in the lateral cavity, and may be used to simultaneously resect the femoral condyles. In using two cutting elements simultaneously, the cutting elements may be linked together by a hinge mechanism 65 to further maintain the cutting elements in a common plane whi le preparing the femoral condyles (Reference is made to Figures 32 and 33).

Thus, for example, in knee surgery, the tool may be mounted to the tibia with the sculpting surface of the tool in engagement with a condylar surface of the femur, that is, one or both of the condyles. As the knee joint is articulated (flexed), the sculpting tool appropriately sculpts the articular surface of the femur in a manner that is dependent upon the individual physiology of that patient's knee, that is, upon the collateral ligaments, the patellar tendon, etc. Although the tool is described in the context of a total knee replacement, it is understood that it has application throughout orthopedics where the surfaces of an articulating joint are to be modified or resurfaced to restore function and relieve pain including but not limited to unicondylar knee replacement or allograft joint surface replacement.

Preferably, the knee joint capsule is surgically accessed without lateral dislocation of the patella, thereby permitting normal flexion of the knee during the sculpting process. The patient's individual physiology and the interplay between the patient's soft tissues and bone work to guide the device used for sculpting cartilage and bone from the end of the femur and/or tibia as they relate to the knee. In the example of the knee, the tibia travels around the end of the femur along a guided path that is controlled by the ligaments and soft tissues that surround and provide support to the knee.

An alternate mount configuration involves an external fixture having burrs attached thereto. The external fixture may be of any configuration that supports the burrs in a position relative to the tibia for sculpting the femur. One example includes an external support member having an arm extending therefrom, the burr attached at the distal end of the arm.

The bone-sculpting tool may be powered by a driving mechanism, for example, a motor. The motor may be an electric motor, a pneumatic motor, or a hydraulic motor integral with the cutting element. Note that in the case of a hydraulic motor, a flexible shaft is not necessary. The cutting element may be driven by available surgical power instruments, such as surgical drills, Midas Rex and Anspaq hi speed drill/c utters, etc. Such equipment is available in pneumatic and battery operated forms. The cutting element may alternately be driven by an electric or pneumatic motor. The power source may also be a hydraulic motor driven by sterile saline solution.

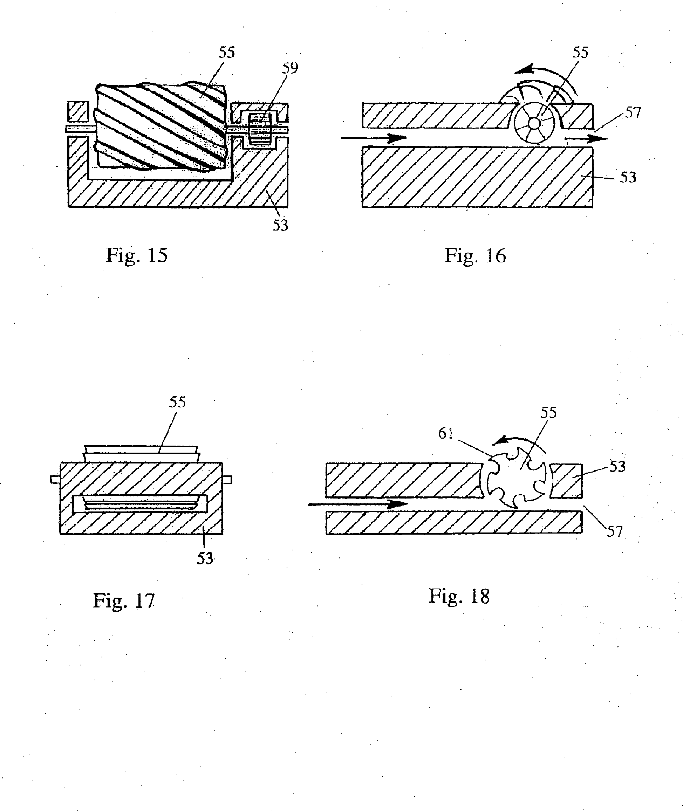

In the case of a hydraulic motor driven with saline solution, the motor may be incorporated into the milling cutter, as illustrated in Figures 15 through 18. The vanes of the hydraulic motor are optionally machined as part of the axel of the milling burr element, or machined into the end face of the milling burr element. Preferably, the housing 53 of the cutting device 55 includes a channel 57 for accommodating saline solution to drive the hydraulic motor. Figures 15 and 16 show a cutting device wherein the vanes of the hydraulic motor are incorporated into the wheel 59 at the distal end of the housing 53. It is also possible, as seen in Figures 17 and 18, to have the blades 61 of the cutting element 55 function as the vanes of the hydraulic motor in which case the saline solution is directed against the cutting element to force rotation.

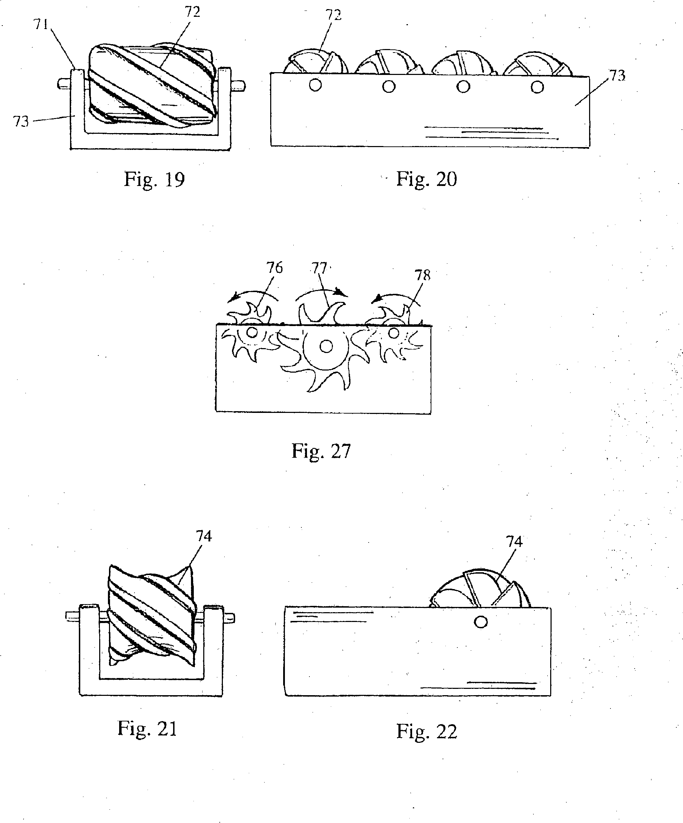

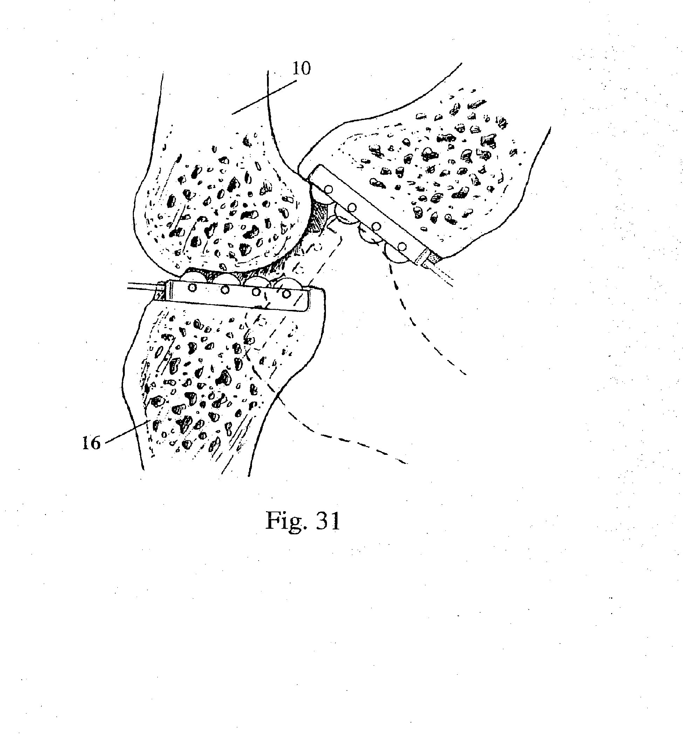

Figures 19 through 30 depict cross -sectional views of various cutting elements that may be used. Figures 19 and 20 show an end and side view, respectively, of cutting element. Milling burrs 72 are placed in the mount 73 and orientated with the axels in a medial to lateral direction. In Figure 31, multiple milling bu rrs are shown to provide contact with the femoral condyle as the knee is flexed and the tibiofemoral contact point moves distally. Alternately, one milling burr may be placed in a position such that it remains in contact with the femoral condyle throughout knee flexion. Although only the options of one or four milling burrs are depicted, one or more milling burrs may be supported in the cradle. Further, the cradle may be provided with shoulders 71 having skidding surfaces for co ntacting the femoral condyle.

Figures 21 and 22 show an end view and a side view, respectively, of an alternate cutting element in which the milling burr 74 is contoured to provide a contoured resection in the femoral condyle. A contoured resection removes less bone and the bone remaining is generally stronger than bone deeper in the condyle.

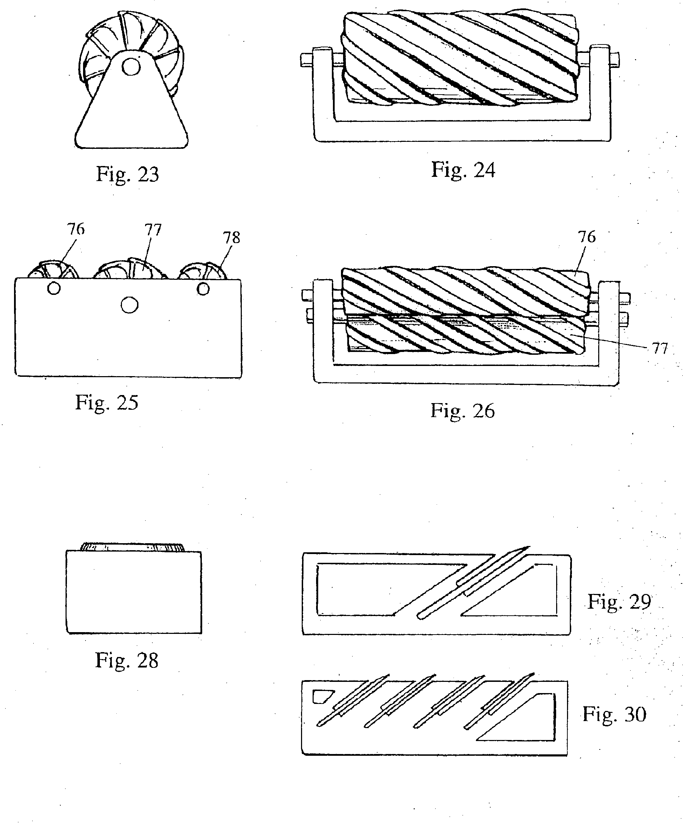

As shown in Figures 23 and 24, another milling burr 72 is oriented with its axel in an anterior to posterior direction. At knee ex tension, the tibiofemoral contact point is near the anterior end of the milling burr. As the knee is flexed, the contact point moves posteriorly and approaches the posterior end of the milling cutter.

In similar fashion, Figures 25 and 26 show three milli ng burrs 76,77, and 78 in parallel with orientated in an anterior to posterior direction. Such an orientation provides for a broad resection of the femoral condyle in one pass or flexion of the tibia. The medial and lateral milling burrs 76 and may be of sm aller diameter than the central milling burr 77, as seen in Figure 27, to provide a smaller corresponding radius between the sidewalls of the cavity created in the femoral condyle and the floor of the cavity.

Cartilage and bone of the femoral condyles may be removed in one or more passes of a shaving element 80 as shown in Figures 28 through 30 The shaving element 80 is off set from the surface of the mount 81 so that a pre -determined amount of bone is shaved off of the femoral condyle with each pass or fl exion of the tibia. One or more shaving elements may be supported in the base of the cutting element.

Using the instrumentation shown, the articular surface of the femur may be sculpted according to the patient's individual physiology by articulating the tibia with reference to the femur. The method involves providing the apparatus having a bone sculpting tool attached to a bone mount, attaching the mount rigidly to the second bone with the tool in bone sculpting engagement with the articular surface of th e first bone, and then sculpting the articular surface by articulating one of the bones with respect to the other. Figure 31 illustrates the kinematics of the articulation of the tibia 16 about the femur 10. The bony resections of the medial and lateral fe moral condyles are made by securing the cutter to the tibia and articulating the tibia. The movement of the tibia in reference to the femur follows a J -curve because of the four bar linkage of the anterior and posterior cruciate ligaments, when both are in tact. In the absence of one or both cruciate ligaments, the movement of the tibia as the knee is flexed is controlled by the collateral and capsular ligaments. The bony support surface thus created in the medial and/or lateral femoral condyles will be shap ed and positioned relative to the kinematics of the given patient.

Preoperative evaluation of patient x -rays may be used to assess deformity of the joint and appropriate spacing required to realign the joint. Additionally, spacers, for example balloons, may be used preoperatively to assess the range of motion of the joint and patient kinematics.

During the surgery appropriate spacers are placed between the bone structures to provide appropriate distraction and alignment of the joint. A distraction force provided between the femur and the tibia during the sculpting procedure may be used to account for material that has worn away from the articular surfaces. Use of a distraction force generally re -establishes normal alignment of the joint. Such spacers also tension the soft tissue structures to reduce the envelop of motion between the bone structures and increase transverse and rotational stability of the joint. The spacer may further be used to support the bone -cutting element during resection of the bone structures. Ligament releases necessary to restore appropriate limb alignment and ligament tension/balance may be performed prior to inserting the spacers.

Any one of a variety of devices may be used to maintain appropriate tension of the ligaments capsule and tendons. Such tensioning devices may include, but are not be limited to, gravity with the weight of the lower limb, intra -articular spacers, bladders, balloons, bellows, gear mechanisms, scissor mechanisms, other expandable devices or other elements th at might engage or attach to the opposing sides of the joint. Moreover, the distraction force may be provided by an expanding base in the cutting element. A distraction device may also be useful in conjunction with a mount having skid surfaces on the shoulders. The shoulder allows the depth and shape of the femoral resection to be controlled both by the articulation of the tibia relative to the femur and the shape of the femur.

Specifically, for pre -operative assessment, spacers such as balloons may be provided in both the medial and the lateral resections. During surgery, a balloon may be provided in the medial resection and a spacer, for example a bellows, having a cutter attached may be provided in the lateral resection. Alternately a bellows having a cutter attached may be provided in both the lateral and the medial resections.

Figures 32 and 33 provide closed and open depictions, respectively, of two cutting elements 61 and 63 linked by a hinge mechanism 65 to maintain the cutting elements in a common plane while preparing the femoral condyles. The hinge mechanism 65 allows adjustability of the placement of the two cutting elements 61 and 63 in reference to one another.

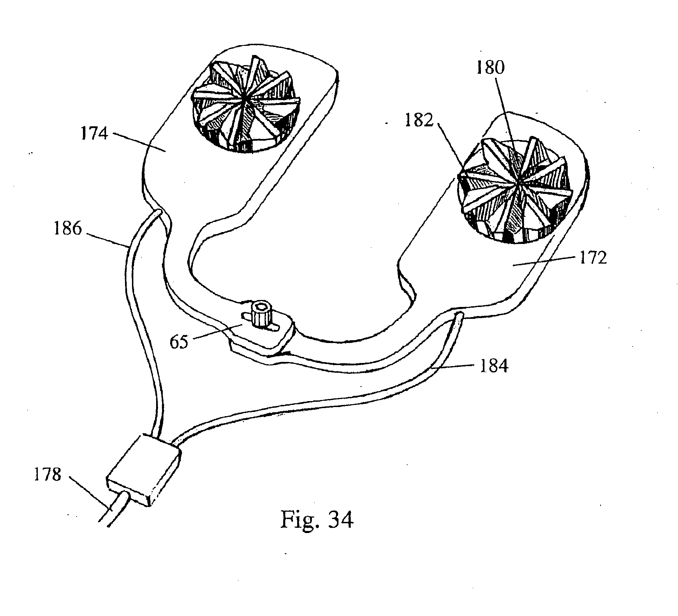

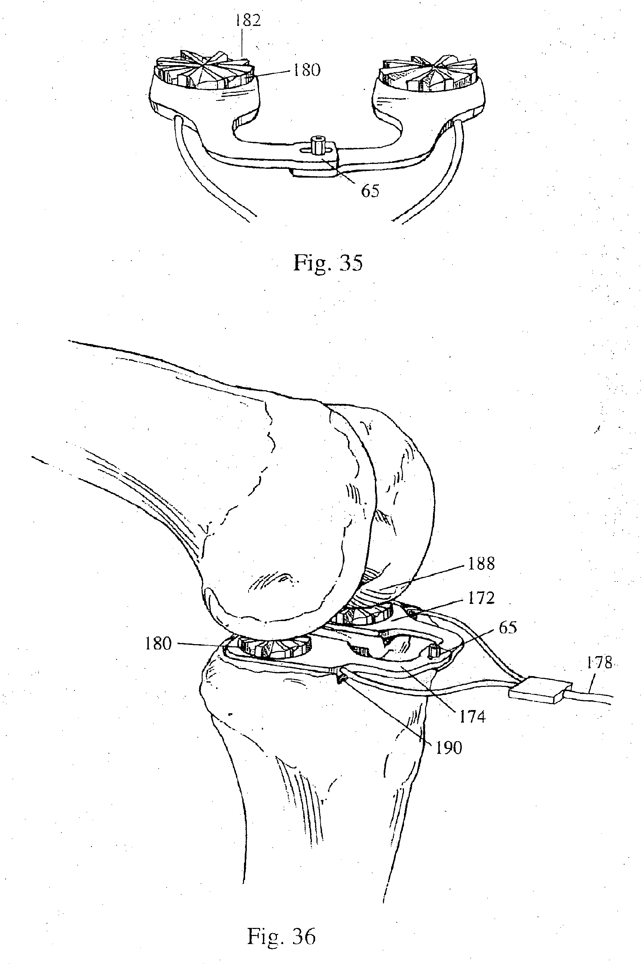

After the creation of resections in the tibial plateau, the femoral sculpting tool of Figure 34 may be placed into the recesses in the tibial plateau. The hinge mechanism 65 enables adjustment of the tool arms 172 and 174 in the medial to lateral direction to accommodate the spacing of the tibial plateau resections and holds the tool arm s 172 and 174 in a common plane to ensure proper orientation of the femoral condyle resections with respect to one another. Cutting elements 180 are mounted into the tool arms 172 and 174 and are driven by a driving mechanism, for example, a pair of gear mechanisms. Preferably, the gear mechanisms are driven by a common flexible drive cable 178 that drives a gear box providing torque to secondary drive cables 184 and 186. As shown in Figure 35, the surfaces of the cutting elements 182 are roughened, or have cutting flutes, to provide cutting of the femoral condyles.

As shown in Figure 36, the knee femoral sculpting tool may be placed in the resections made in the tibial plateau 190. The cutting discs 180 are in contact with the femoral condyl es 188. The knee is flexed resulting in relative motion of the femoral condyles across the cutting discs, thereby resecting the medial and lateral condyles of the femur at the same time. Soft tissue structures spanning the knee guide the cutting motion along the normal kinematic motion of the given knee joint. The cutting discs 180 rotate in a transverse plane.

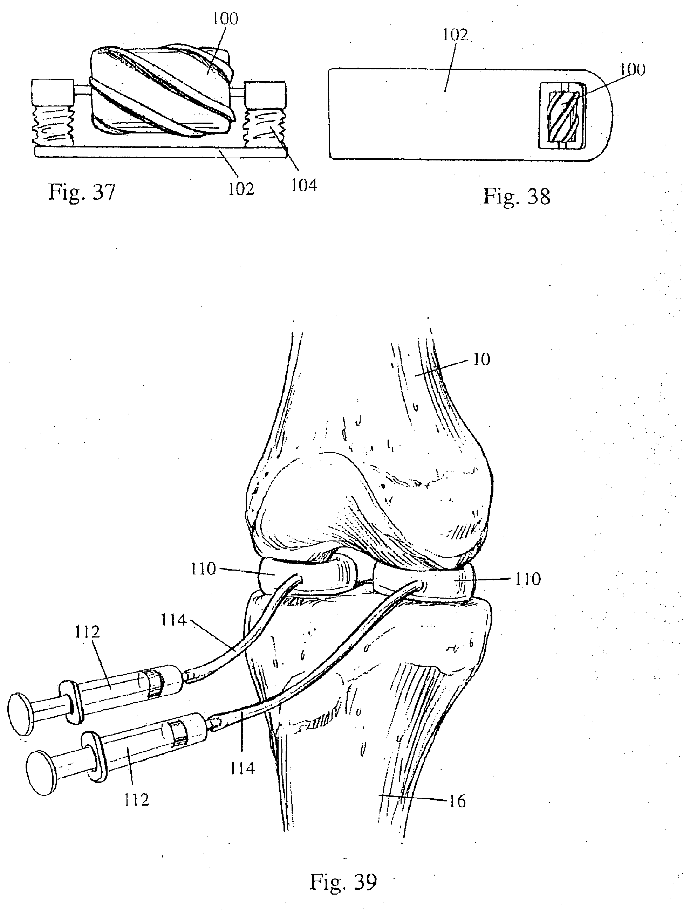

Figures 37 and 38 provide end and top views, respectively, of a cutting element 100 supported in a platform 102 that is configured for elevation v ia fluid pressure applied to a distractor 104 that surrounds the cutting element 100. Applying pressure to the distractor 104 forces the milling burr into the femoral condyle to a predetermined depth as set by the top surface of the cutting element. The distractor 104, in combination with the top surface of the cutting element, ensures proper resection depth while tensioning the soft tissue structures spanning the knee joint. The benefit of tensioning the soft tissue structures is to reduce the envelop of motion of the knee, stabilize the knee and provide increased accuracy and repeatability of the femoral condyle resections. Alternatively a spacer may be placed between the floor of the cavity created in the tibia and the bottom of the cutting element to provide a distraction force.

Figure 39 shows balloon spacers 110 used to support the femoral condyles to distract the femur 10. Syringes or pumps 112 may be attached via hoses 114 to balloon spacers 110. Balloon spacers 110 are an example of an expandable spacer. Where an expandable spacer is used, pre -operative evaluation should be performed. During surgery an expandable spacer is placed between the bone structures to be resected. The cutting element may be housed in the dynamic spacer with th e cutting element adjustable to the dynamic spacer to set the depth of resection. The dynamic spacer may function under load control in which case a constant distraction force is applied between the bone structures throughout a range of motion, or under displacement control. Under displacement control, a constant displacement is maintained between the bone structures throughout a range of motion. In each case, the dynamic spacer houses the cutting element and the cutting element is held at a pre-set depth relative to the bone structure being resected while the joint is flexed and extended. The dynamic spacer allows the kinematics of the joint to define the resection path in each of the bone structures.

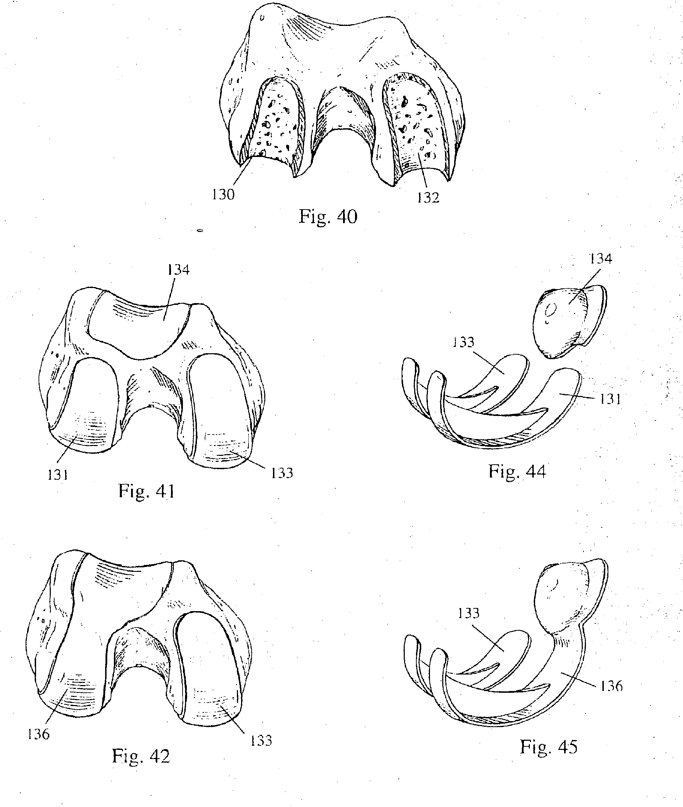

As the tibia is articulated through flexion and extensi on, the femoral cutter prepares resections in the femoral condyles for receiving femoral components of a knee implant. Figure 40 shows the bone resections 130 and 132 in the lateral and medial femoral condyles respectively. The patellar groove may be prepared in a similar fashion (not shown) with a femoral cutter secured to the patella. Figures 41 and 42 depict alternate bone resections with representative implants placed in the femoral condyle as may be desired.

Implants. The surgical procedure is preferably performed through minimally invasive incisions that do not necessitate subluxation or dislocation of the patella. Therefore, implants such as the femoral, tibial or patellar implants are designed that may be fit through minimally invasive incisions and either oriented or joined within the joint. The femoral and tibial implants may be attached to bone with conventional bonding methods such as, but not limited to, polymethylmethacrylate, or by direct attachment to bone as with, but no t limited to, a porous ingrowth surface.

The tibial baseplate is optionally configured as one piece to cover most of the prepared surface of the tibial plateau as relates to the knee. If configured as a single platform, the tibial baseplate provides a capture mechanism for a fixed bearing or a mobile bearing insert for either the medial or lateral tibiofemoral compartment. As an option, a single platform is designed that provides a fixed bearing capture mechanism for the medial compartment and a mobile bea ring capture mechanism or a simple platform to receive a mobile bearing insert for the lateral tibiofemoral compartment. Since right and left tibial baseplates are required, the same baseplate may be used for a mobile bearing medial insert and a fixed bear ing lateral insert.

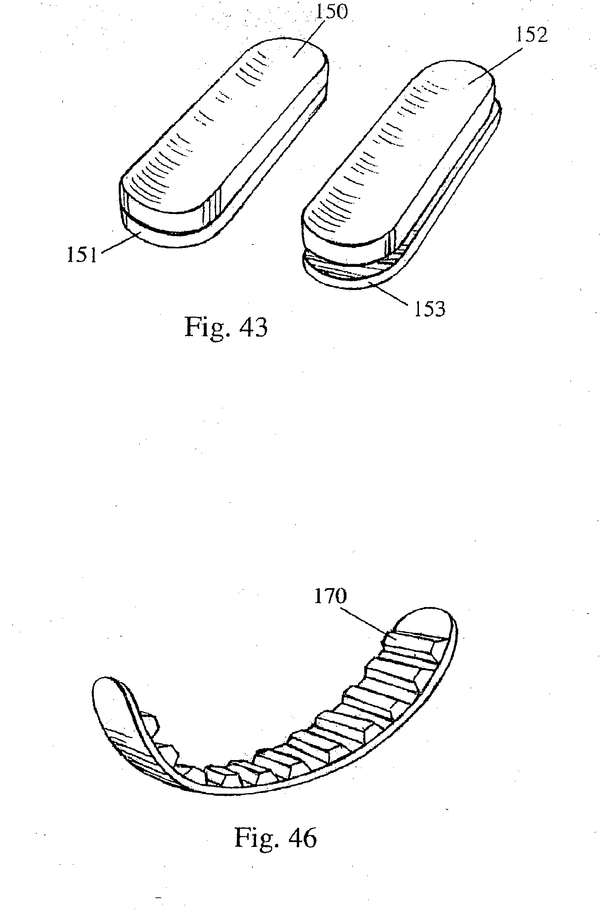

Alternatively, as depicted in Figure 43, the tibial implants may be configured as separate plateau baseplates for the medial and lateral compartments. These platforms might be oriented one to the other by an alignment instrument that dictates their orientation in relationship to each other and/or to the femoral components. The tibial baseplates may be fixed bearing and manufactured completely of polyethylene. Thus, fixed bearing tibial components 150 with a metal support tray 151, and mobile bearing tibial components 152 with a metal support tray 153 may be used in the same knee replacement surgery. Furthermore, the tibial baseplate may accommodate separate fixed and mobile bearing inserts in either or both medial and lateral compartmen ts.

It is preferable to place all of the implants through small incisions. As seen in Figure 44, the femoral implants include a first component 133 to resurface the articulating surface of the medial condyle and a second component 131 to resurface the articulating surface of the lateral condyle. An optional third component 134 may be provided to resurface the femoral side of the patellofemoral joint. The convex surface of the femoral condyle is the bearing surface and interacts with the tibial bearing implants. Optionally, the femoral component (s) may include a fin along its support or convex internal surface for upward driven implantation. The fin may be shaped as a web extending from one portion of the internal surface to another.

As shown in Figure 42, the lateral femoral implant may be continuous with the patellar flange forming a unitary piece 136 that may be passed through a small incision. To accommodate the continuous piece, the lateral condylar component and the patellofemoral component optionally may be a single component 136 extending from the top of the patellofemoral groove and extending over the lateral condyle both distally and posteriorly, as seen in Figure 45. Figure 45 provides a side view of a femoral implant combining the lateral condylar component and the patellofemoral component into a single component 136.

The bearing elements may be manufactured of ultra high molecular weight polyethylene but may also be manufactured of any suitable biocompatible material as known in the art. The bearing elements generally include three compartments: medial tibial condyle, lateral tibial condyle and patella. Preferably, a choice of bearing elements is provided for either fixed or mobile bearing of each compartment. Thus, for example, the surgeon would have at his discretion inserting either a mobile bearing or a fixed bearing insert into each of the tibial components, one medial and one lateral.

The femoral components may include an alignment device to orient separate femoral components in relationship to one another and/or to the tibial components. Surgical navigation may be used in concert with bone preparation and component orientation.

The femoral components are provided in a variety of sizes and optionally include components that are flexible t o provide optimum fit for minor variations in the shape of the prepared femoral condyles.

Figure 46 is an illustration of femoral condyle implants configured as flexible implants. The outer surface of the femoral condyle implant is a thin sheet of metal forming an articular surface, preferably of cobalt chromium alloy. Other suitable implant grade alloys, polymers, or metals, for example, stainless steel, titanium alloy, or Nitinol, may be used. In order to provide uniform deflection in one plane, the implant is thin and of uniform cross section. The support surface of the femoral condyle implant is lined with molded bone cement, such as polymethylmethacrylate (PMMA or PMA), and spacers that are bonded to the articular surface. The spacers may be shaped as blocks or any other configuration suitable for molding in place during fabrication. Generally, the spacers are shaped to span the femoral condyle implant from side to side, in a coronal plane, while providing spaces between spacers at given intervals to facilitate mild flexing of the articular surface. Such flexing enables the flexible femoral condyle implant to conform to the unique shape of the prepared bony support surface in the femoral condyle, thereby taking full advantage of the kinematicly defined support surface. Such implants are provided in a range of sizes to accommodate individual patient physiology and to minimize the amount of flexing a given implant may make in conforming to the prepared surface. Hence, the distortion of the articular surface is minimal.

In use, the resected femoral condyle is covered with doughy bone cement. The femoral implant is placed and loaded against the resected femoral condyle until the bone cement cures.

The preferred method for preparing the femoral condyle uses the tibia as a support for the milling cutter. The soft tissue structures of the knee provide the path of motion to move the cutter through the femoral condyle. The kinematics of the knee are well understood and defined. This approach necessarily results in a unique shape machined into each femoral condyle due to variations in soft tissue structures and bony structures from patient to patient.

Alternatively, the femoral condyles may be ridged and of given size. Each implant is composed of a plurality of components 170. The components 170 are cemented in place with bone cement, which acts as a grouting material to fill the space between the implant and the supporting bone. Bone cement has been shown to provide long term implant stability when applied in thicknesses up to two millimeters. Hence, a range of implant sizes covers the range of femoral condyle sizes anticipated and the variation in shape anticipated.

Hip Joint Anatomy and Surgical Approaches . Figure 47 illustrates the general anatomy of the hip joint and a typical surgical approach 200 to the hip joint to expose the proximal femur 202 and the 204. In traditional total hip replacement there are generally four surgical approaches to the hip joint. These include posterior approaches without trochanteric osteotomy, trans -trochanteric approaches, anterior approaches without trochanteric osteotomy, and Smith - Peterson approaches. Such approaches are described in detail in various orthopedic reference texts such as "Operative Orthopedics" by M. W. Chapman, MD, J. B. Lippincott Company, 1988. In addition, a direct lateral approach is commonly used for total hip arthroplasty. The most common surgical approach to the hip is posterior, and the musculature disrupted may include the short internal and external rotators, tensor fascia femoris, quadratus femoris, piriformis, and on occasion part of the gluteus medius and minimus, and the gluteus maximus.

In minimally invasive total hip surgery, the incision 206 is typ ically 6 cm as shown in Figure 48. While 6 cm, or 2 -4 inches, is a typical length for a minimally invasive surgical incision, there may be some variation due to patient physiology, surgeon preferences, and/or other factors. The surgical approach involves separating the gluteus maximus muscle through blunt dissection to gain access to the hip joint capsule and the trochanteric fossa. Muscle disruption is usually limited to release of the piriformis tendon at the trochanteric fossa. It should be noted that th ere are variations to the surgical approaches described that are known to someone skilled in the art.

Figure 48 illustrates a minimally invasive surgical approach to the hip joint. The general approach is posterior, and the musculature disrupted includes release of the piriformis tendon. The incision is just large enough to expose the femoral head and acetabulum, and to enable placement of a hemispherical reamer 208, drive mechanism 210, and femoral broach 212.

In contrast to the minimally invasive technique provided, a total hip replacement surgery involves exposing the hip joint through a large incision to provide the surgeon full visualization of the hip joint and the region and access for surgical power instruments. The femoral head is removed and the femoral canal is reamed and broached to prepare the bony surface to support the hip stem. The stem may be cemented in place or press fit for bony ingrowth. The acetabulum is prepared, most typically using a hemispherical reamer attached to a surgical hand drill to remove cartilage down to bleeding bone. The surgical exposure as shown in Figure 52 generally ranges between eight and twelve inches in length and may result in extensive trauma to the soft tissues surrounding the hip joint.

Minimally Invasive Acetabular Reamer System (MIAR) As seen in Figure 49, the MIAR of the present disclosure, for use with hip replacement surgery, is either a modular or non-modular construct comprising a femoral trial 216, a drive mechanism 218 (either integral or separate) and a hemispherical reamer 220 or similar device for removing cartilage and bone from the acetabular fossa. The hemispherical reamer 220 or similar device includes an attachment component (not shown) for attaching either to the femur, directly or indirectly, or to a mount that itself is attachable to the femur, directly or indirectly. Discussion of the attachment of the MIAR to the femur, directly or indirectly, should be read as broadly encompassing attachment by the reamer directly to the femur (or femora I component) or attachment by the reamer to a mount that is attached to the femur (or femoral component). The reaming system, especially as a modular construct, enables placement of the components through a small incision and minimizes the number of components in the instrument set. In the minimally invasive procedure, the proximal femur does not have to be displaced during acetabular preparation as is necessary with conventional hip arthroplasty. Therefore, the procedure requires only a minimal release of muscles and tendons and, consequently, minimal trauma to muscles and tendons that attach to the proximal femur. Although the MIAR is described in the context of a total hip replacement, it is understood that it has application throughout or thopedics where the surfaces of an articulating joint are to be modified or resurfaced to restore function and relieve pain. The MIAR system uses a drive mechanism anchored to or mounted on a device such as a reamer, broach, or other suitable device that i s secured to one bone and, with the joint reduced or placed in position of reduction, may be activated to prepare, with a hemispherical reamer or suitable bone sculpting tool, the opposite side of the joint to receive artificial components.

With reference to the hip joint, the femoral head is removed either before or after the femoral canal is reamed and broached to prepare a bony surface to support the hip stem or broach to be inserted. The minimally invasive acetabular reamer is mounted to the broach, re amer, trial femoral component or other device inserted into the proximal femur. It is possible to attach the MIAR directly to the proximal femur, however the instruments and the femoral implant provide an advantageous support structure as these instruments , such as rasps, broaches, trials or the implant, conform closely to the prepared bony surface and provides a rigid metal structure to which the MIAR may be mounted. Therefore, preferably, the MIAR is directly or indirectly attached to the femoral broach that is secured within the proximal femoral canal. It is noted that throughout the description, rasps, trials, broaches, implants and stems are used interchangeably in relation to the system. Alternatively the MIAR may be attached directly to the femur, the femoral trial or the femoral implant. With the MIAR directly or indirectly attached to the femur, the reamer head is placed into the acetabulum. The MIAR is activated to initiate cartilage and bone removal as the femur is positioned. The operating surgeon controls the MIAR by placing and/or moving the leg as necessary to create a spherical reaming of the acetabulum. Surgical navigation may be utilized.

The femoral trials are available in an array of sizes to accommodate the size range of the proximal femur. The hemispherical reamers are available in a range of diameters to accommodate the size range of the acetabulum. Preferably, the drive mechanism is interchangeable amongst the femoral trails and amongst the hemispherical reamers. Alternatively there is a drive mechanism for each femoral trial or groups of trials. The trials may be grouped by size, or by right and left. The example given is for the MIAR attached directly or indirectly to a femora I rasp. Similar combinations are possible when the drive mechanism is directly or indirectly attached to a femoral trial or femoral implant.

An example of a procedure includes the following steps: the appropriate femoral trial is placed into the prepared proximal femur; the drive mechanism is placed onto the proximal aspect of the femoral trial followed by placement of the appropriate sized hemispherical reamer onto the drive mechanism; the hip is reduced and the reaming system is activated to prepare the acetabulum. Of course, if the MIAR is not modular, it is placed as a unit, the hip is reduced, and the reaming system is activated.

As shown in Figure 54, the acetabular reamer 220, which is provided in a range of sizes, attaches to the drive mechanism 218 at the support plate 235, which provides quick attachment to the drive mechanism 218. The reamer is preferably rigidly supported on the femoral side such that sufficient stability is provided to prevent relative motion between the MIAR and the femur during articulation. Such stability is generally provided through the placement of the broach 216, femoral trial or femoral implant in the femoral canal. Figure 49 illustrates a MIAR.

Support for the MIAR is provided by a femoral broach 216. The drive mechanism 218 is supported by the femoral broach 216. Figure 49 further shows the drive shaft 240 of the drive mechanism 218 supported in the drive mechanism housing, which is supported by the femoral broach 216.

As shown in Figure 51, the drive mechanism 218 may use a worm 238 and worm gear 236 combination, bevel gears, spur gears, belts or chain drives or other suitable mechanism to transfer rotation or oscillation to the acetabular reamer. In Figure 50, a worm gear 236 is attached to the drive shaft 234 which in turn is driven by a worm (behind the worm gear). A worm and worm gear combination represents only one possible drive mechanism that may be used to drive the ace tabular reamer and is intended to be illustrative but not limiting. Any other drive mechanism known to those skilled in the art may be used. Figure 51 depicts the worm 238 supported by an input drive shaft 240.

As shown in Figure 53, a flexible drive cable 228 is attached to the drive shaft 240. Optionally, a sleeve mounted to the drive mechanism housing may extend through the surgical incision and contain the drive shaft 240 with the flexible cable 221 attached outside of the su rgical incision. Torque generated by the drive mechanism is reacted between the drive mechanism and the femoral trial by a rotational stop 242. (See Figure 8.)

The acetabulum is prepared by rotating or oscillating a hemispherical reamer within the acetabu lum. The hemispherical reamers may be reamers, cutters, or other devices used for removing cartilage and bone from the acetabular fossa. Alternatively, non-mechanical cutting instruments such as lasers, water jet cutting, ultrasonic probes, chemical or oth er devices to remove tissue can be used. Such devices involve rotation or oscillation of the reamer with the device supported by the femur. As shown in Figure 52, the MIAR may be self -contained with an internal power source to drive the reamer, or may have an external power source to drive the reamer. Likewise, the motor 222 may be internal to the drive mechanism or may be external with torque transferred to the drive mechanism via appropriate shaft or connection. The drive mechan ism may be constructed of mechanical components such as gears, cams, levers, belt and pulleys or chains. Power sources for the drive mechanism to drive the reamer include fluid to drive a hydraulic motor, gas to drive a pneumatic motor, electrical to drive an electric motor (either integral to the femoral trial or via flexible drive cable connecting the motor to the drive mechanism), solenoid or other suitable power source to provide rotation or oscillation to the reamer. Alternately, the drive mechanism may be driven by available surgical power instruments, such as surgical drills, Midas Rex and Anspaq hi speed drill/cutters, etc. Such equipment is available in pneumatic and battery-operated forms. Preferably, the drive mechanism is driven by an external power source transferring torque through a flexible drive shaft. Alternatively, the power source may be housed within the femoral trial or broach.

Alternatively, the drive mechanism may be configured for use with any one of the attachment mechanisms provided by various manufacturers of total hip systems to attach trial necks to femoral trials. The attachment thus may be a peg in groove, a peg in hole, a conical taper, a screw fit, or a threaded attachment. Preferably, the drive mechanism is designed to attach to a femoral trial or rasp/trial provided with the total hip system with which the MIAR is being used. The proximal surface of the drive mechanism is designed with a quick attach mechanism that fits an array of acetabular reamer sizes.

Alternatively the drive mechanism is supported by the femoral taper that supports the femoral head implant or implant trial. The femoral stem trial is placed into the prepared femoral canal and the appropriate femoral neck trial is pl aced onto the stem trial. The drive mechanism is placed onto the femoral neck trial taper and the appropriately sized reamer is directly or indirectly attached to the drive mechanism. Optionally, the femoral stem trial and femoral neck trial may be integrally formed. In this approach, the femoral canal is prepared and the appropriately sized femoral stem is selected based on the patient's femoral anatomy. The femoral stem implant is placed into the prepared femur and the drive mechanism with appropriately sized acetabular reamer is placed onto the implant to prepare the acetabulum.

Alternatively, the drive mechanism may be integral to the femoral trial or the acetabular reamers. The hemispherical reamers are modular and allow changing reamer sizes during the procedure. As seen in Figure 52, in surgical use, the appropriate femoral broach 216 with integral drive mechanism, in this case a hydraulic motor 222, is placed into the prepared proximal femur and the appropriately sized hemispherical reamer 220 is directly or indirectly attached to the broach via the drive mechanism. The trial stem includes a drive mechanism 222 that is housed within the proximal aspect of the broach 216. The drive mechanism 222, which may be a hydraulic motor within the broach, rotates the drive shaft 226 and support plate 235 which in turn rotate the acetabular reamer 220 to prepare the acetabulum. Alternatively, as seen in Figure 53, the appropriate acetabular reamer 220 with integral drive mechanism 218 is placed into the acetabular fossa 232 and directly or indirectly attached to the femoral trial 216. Acetabular preparation is performed with the hip joint articulated (reduced).

Figures 49, 50, 51, 53 and 54 illustrate the mechanical drive mechanism used in one MIAR system. Figure 53 shows the MIAR placed into the proximal femur 230 with the hemispherical reamer 220 in contact with the 232. Figure 54 illustrates an exploded, view of one MIAR system. The drive shaft 234 extending distally from the drive mechanism 222 passes into receiving hole 254 to attach the drive mechanism 222 to the broach The anti -rotation pin engages the receiving hole 256 to add stability and rotational resistance between the drive mechanism and broach. The re amer 220 attaches to a support plate 235 that Is part of the drive mechanism 218. The surface 258 of the reamer 220 conically locks to the support plate 235.

Yet another acetabular reamer is assembled in a collapsed state to allow ease of reduction of the hip joint with the MIAR system in place. The reamer is elongated from the femoral housing or from the drive or gear mechanism of the This elongation may be accomplished by a variety of devices, for example shim plates, spacers or other suitable device placed between the elements. Alternatively the MIAR may be elongated by means of pneumatic pressure, lead screws or other power sources. The manner by which the MIAR is elongated is not critical and any suitable device or me thod may be used. When sufficient resistance is encountered by the joint capsule and/or other soft tissue elements about the hip, the MIAR is activated to initiate acetabular bone preparation. The process of acetabular reaming is enhanced by pressure created through tensioning the soft tissue elements. In the example of using pneumatic force, gas pressure first elongates the MIAR construct. After a specified amount of resistance is encountered to elongation, pneumatic pressure is transferred to elements that generate torque to turn the acetabular reamer.

Minimally Invasive Acetabular Impaction System. Once the acetabulum has been prepared, an acetabular implant is secured to the supporting bone, usually by either bone cement or press-fit. In the case of a cemented acetabular component, the bone surface is oversized relative to the implant size. The bony surface and the implant are covered with bone cement. The implant is then placed into the acetabulum and pressed into position forming a uniform layer of bon e cement between the acetabular component and supporting bone. In the case of a press fit acetabular component, the bone surface is line -to-line or slightly undersized relative to the implant size. The implant is impacted into place in the supporting bone. In standard total hip surgery, a straight handled impactor is commonly used to impact the acetabular component. The extensive exposure typically used in traditional total hip surgery provides the clearance to align the impactor relative to the acetabulum. However, in the case of a minimally invasive total hip replacement, the incision is too small to allow proper orientation of a standard straight handled impactor. Use of a standard impactor requires making a second incision to pass the impactor through muscle and tissue in the correct orientation relative to the acetabulum. The acetabular component must be positioned properly to provide normal function and to prevent dislocation of the hip joint. Making a second incision and disrupting more muscle is contr ary to the goal of a minimally invasive procedure. Therefore, a device that impacts the acetabular component through a minimally invasive incision is needed. In one embodiment, the current invention includes a device designed to directly or indirectly att ach to the femoral trial and provide an impaction force to properly seat the implant. A variety of components and methods for placement thereof may be used. Example components for implanting in the acetabulum include, but are not limited to, cemented shell s or press fit cups.

As seen in Figure 56, the impaction device preferably includes a pneumatic impaction hammer 260 to the femoral broach 216 and an optional attachment component for attaching to the shell 252 of the component. The impaction device 260 and acetabular component 252 may be placed into the surgical site independently and assembled in the operative site. Alternatively, the impaction device 260 and acetabular component 252 may be assembled prior to placing the impaction device onto the broach 216. With the acetabular shell directly or indirectly attached to the impactor and the impactor secured to the femoral broach, the shell is placed into the acetabulum by reducing the hip joint. The broach, femur and mass of the leg serve as counter weight s to counteract the force of the impaction device. An additional counter weight may be directly or indirectly attached to the impaction device via a connection shaft extending outside of the incision and attaching to a weight or an external resistance to impaction forces.

The impaction device may be powered by a pneumatic impaction hammer, a hydraulic piston, a linear actuator or solenoid, an electromechanical device, a spring activated device or any other suitable force generating mechanism. The power sou rce may originate outside of the operative site or may be integral with the impaction device. As an alternative, a hand held impactor with a handle angled to allow access through a minimally invasive incision may be used to impact the acetabular component. In a preferred embodiment, the impactor is a single ended air driven piston and cylinder as shown in Figure 57. The back face 292 of the impactor housing 268 is configured to attach to the broach previously described. Within the housing is a primary pist on 282 that travels in a primary cylinder 294. In its retracted position (shown) a push rod 286 of a secondary piston 280 engages a retaining groove 288 in a primary piston 282. The secondary piston 280 is held in an extended position by a secondary spring 278. Air pressure is applied via a primary tube 284 to the back of the primary piston 282 to charge the system. The primary piston 282 is held in place by the push rod 286 of the secondary piston 280. Air pressure is applied to the secondary tube 276 to p ull the push rod and the secondary piston 280 out of the retaining groove 288 in the primary piston 282, thereby releasing the primary piston 282 to impact the top surface of the cylinder 298. The impaction force is carried through the impactor housing 268 and delivered to the acetabular shell (not shown) via a cup adaptor 264. The cup adaptor 264 has a threaded end 290 that engages the acetabular shell. The other end of the cup adaptor 264 has a box shaped recess 300 that fits over a mating prominence 270 on the top surface of the impactor housing 268.

After an impaction cycle, the pressure to the primary tube 284 is released and the primary piston 282 is forced back into a retracted position by a return spring 274. When the primary piston 282 is in its retracted position, the air pressure to the secondary tube 276 is released and the secondary piston 280 is pushed back into locked position by a secondary return spring 278. Pressure is applied to the primary tube 284 to charge the impactor and the cycle i s repeated.

In surgical use, the cup impactor 260 and broach may be assembled outside of the surgical site, then placed into the prepared proximal femur. Alternatively, the broach may first be placed into the proximal femur, then the cup impactor 260 attached to the broach. With the cup impactor 260 in place, the cup adaptor 264 is attached to the cup implant and the recess 300 in the adapter is placed over the mating prominence 270 on the top of the cup impactor. The hip joint is reduced, placing the acetabular shell into the acetabulum. An alignment guide (not shown) is attached to the cup impactor to aid the surgeon in properly orientating the shell with respect to the pelvis. Alternatively, a surgical navigation system may be used to position the acetabular shell by referencing the cup impactor and the acetabulum. Once in position, the shell is impacted into the acetabulum by triggering the cup impactor with successive impactions. In a preferred embodiment, the trigger releases one impaction, then the cup impactor resets for a further impaction as necessary. In an alternate embodiment, the trigger releases continuous impactions for the duration that the trigger is on.

Of course the impaction device is suitable for use in placing an implant other than an acetabular component. The impaction device may be used for seating an implant in a second bone in any joint replacement wherein the implant may be placed on the impaction device, aligned with a second bone, and force imparted to the implant, the force bei ng reacted with the first bone and the second bone.

A typical surgical procedure for the MIAR is as follows: Using the instrumentation shown, the articular surface of the acetabulum may be sculpted according to the patient's individual physiology by articulating the femur with reference to the acetabulum. The method involves providing an apparatus having a bone sculpting tool directly or indirectly attached to a bone mount such as a femoral trial stem, attaching the mount rigidly to the femur with the tool in bone sculpting engagement with the and then sculpting the by articulating the femur with respect to the joint.

The hip joint is a ball in socket joint, hence rotation of the femur while supporting the in a spherical preparation of the acetabulum. Alt ernatively, the MIAR, having a suitable reamer and drive mechanism, may be placed into the acetabulum to remove bone without rotating the femur.

Preferably, the trochanteric fossa is surgically accessed with minimal disruption of muscle and tendon insertions to the trochanter and surrounding area. The approach may be at the posterior border of the gluteus medius and minimus, anterior in the interval between the sartorius and the rectus, or a direct lateral exposure. The hip may be dislocated posteriorly if a posterior approach is used or anteriorly if either a lateral or anterior approach is used. Alternatively, the hip may remain reduced while the femoral canal is prepared and the femoral neck is resected.

The femoral neck is resected and the femoral head is removed. The resection and removal may be performed with conventional cutting devices such as oscillating saws. The femur is oriented to align the femoral canal with the incision. The femoral canal is prepared using sequential reaming and broaching. Bony preparation is per the technique specified for the particular total hip stem being used and at the surgeon's discretion.

An appropriately sized femoral trial is placed into the femur. The drive mechanism is directly or indirectly attached to the femoral trial. Preferably, the drive mechanism is designed to mount directly onto the femoral trial.

The acetabular reamer is directly or indirectly attached to the drive mechanism. The appropriate reamer is selected by the surgeon. The surgeon may choose to measure the diameter of the removed femoral head as an aid in selecting the most appropriately sized acetabular reamer. The surgeon may choose to use surgical navigation.

The hip joint is reduced and the hip is articulated with the drive me chanism and acetabular reamer in place. Elongation of the MIAR construct is optionally carried out to appropriately tension the soft tissue elements about the hip. The drive mechanism is activated to prepare the acetabulum. If necessary, the femur may be advanced while the hip joint is manipulated to ensure spherical and uniform reaming of the may be used to check the orientation and depth of the acetabular reamer.

At the surgeon's discretion, the depth and uniformity of reaming may be checked periodically during the procedure. This may be done by dislocating the hip, removing the reamer and attaching the illumination and irrigation devices (or a combined illumination and irrigation device) to the femoral trial. The hip is reduced with the illumination and irrigating devices in place and the operative site is cleared with irrigation and suction. The prepared surface of the acetabulum may then be inspected. After inspection, the illumination and irrigation devices are removed and the drive mechanism and reamer are replaced. Alternatively, the depth of reaming may be assessed under imaging of the hip joint.

The articulation of the hip joint to prepare the may be repeated with sequentially larger reamers until the appropriate size is reached. Further, the size and preparation may be checked with the illumination and irrigation devices as necessary. Once the appropriate size is reached, the acetabular reamer and the drive mechanism are removed.

After preparation of the acetabulum, an appropriate acetabular component is implanted. The appropriate acetabular component may be pre -selected or may be selected after surgical preparation of the acetabulum. If the desired component is a cemented cup, the cup is cemented in place.

If the desired component is a press fit cup, a cup impactor is attached to the broach and placed into the prepared proximal femur. Alternatively, the broach may be placed in the prepared femoral canal first and then the cup impactor attached to the broach. The acetabular shell is attached to the cup adaptor and placed onto cup impactor. The hip joint is reduced and the shell is positioned in the acetabular fossa. An alignment guide is attached to the cup impactor to aid the surgeon in proper orientation of the shell during impaction. The cup impactor is triggered, thereby impacting the shell. An alternative technique for placing a press fit cup may use image guided surgery or an alignment device protruding from the incision. The guiding system is used to advance the cup into proper orientation. The minimally invasive acetabular impactor is activated to securely seat the cup into the Regardless of technique, after placement of the press fit cup, the impaction device is removed. Alternatively, a surgical navigation system may be used for positioning, aligning, and monitoring the cup or cup impactor during impaction. Cup monitoring includes real time evaluation of the cup position relative to anatomical landmarks captured by the surgical navigation system after preparing the acetabulum and before placing the cup so as to indicate cup seating and cup alignment.

The acetabular liner is placed into the shell and a trial femoral neck and head are placed onto the femoral trial. The range of motion and hip stability are checked and the appropriate femoral implant is selected. The femoral trials are removed and the femoral component is implanted per manufacturer specifications.

Accordingly, an apparatus and method for minimally invasive sculpting the articular surface of a first bone that normally articulates with a second bone is provided. In one apparatus, a bone sculpting tool is provided on the tibia for sculpting the femur when the tibia is articulated with reference to the femur. In another embodiment, the is sculpted by providing a bone sculpting too I on the femur, aligning the bone sculpting tool with the femur and engaging the surface of the with the bone sculpting tool.

## Claims

1. An apparatus for imparting force to seat an implant (252) in a first bone (232), the apparatus comprising:
an impaction device (260) including an attachment portion (216) for attaching the impaction device to an adjacent second bone (230), the impaction device structured to seat the implant in the first bone by imparting force to the implant, said force being transmitted by said implant to said first bone, **characterised in that**
the reaction to said force is transmitted by said attachment portion to said second bone.

2. The apparatus of claim 1, further comprising an external alignment guide for orienting the implant in the first bone.

3. The apparatus of claim 1, further comprising a surgical navigation system for positioning and aligning the implant with respect to the first bone.

4. The apparatus of claim 1, further comprising a surgical navigation system for positioning, monitoring and aligning the impaction device with respect to the first bone.

5. The apparatus of claim 1, further comprising a mount for attachment to the attachment portion, the mount being adapted for attachment to the second bone.

6. The apparatus of claim 1, wherein the implant is an acetabular shell and the impaction device is configured to seat the acetabular shell in an acetabulum.

7. The apparatus of claim 1 or 6, further comprising an adapter for attaching the implant to the impaction device.

8. The apparatus of claim 7, wherein the adapter restricts the implant from rotating.

9. The apparatus of claim 6, wherein the attachment portion is adapted for direct attachment to the second bone.

10. The apparatus of claim 6, wherein the attachment portion is adapted for indirect attachment to the second bone.

11. The apparatus of claim 1, wherein the impaction device is a pneumatic impaction hammer.

12. The apparatus of claim 1, wherein the impaction device is a linear actuator.

13. The apparatus of claim 1, wherein the impaction device is a hydraulic piston.

14. The apparatus of claim 1, wherein the impaction device is spring activated.

15. The apparatus of claim 1, wherein the impaction device is configured for activation for a single impaction cycle for each activation.

16. The apparatus of claim 1, wherein the impaction device is configured for activation for multiple impactions cycles while activated.

17. The apparatus of claims 1 through 16 wherein the first bone is an acetabulum and the second bone is a femur.

## Patentansprüche

1. Vorrichtung zum Aufbringen einer Kraft zum Platzieren eines Implantats (252) in einem ersten Knochen (232), wobei die Vorrichtung Folgendes umfasst:
eine Einschlagvorrichtung (260) mit einem Anbringungsteil (216) zum Anbringen der Einschlagvorrichtung an einem benachbarten zweiten Knochen (230), wobei die Einschlagvorrichtung so strukturiert ist, dass sie das Implantat durch Aufbringen einer Kraft auf das Implantat im ersten Knochen platziert, wobei die genannte Kraft durch das genannte Implantat auf den genannten ersten Knochen übertragen wird, **dadurch gekennzeichnet, dass** die Reaktion auf die genannte Kraft von dem genannten Anbringungsteil auf den genannten zweiten Knochen übertragen wird.

2. Vorrichtung nach Anspruch 1, die ferner eine externe Ausrichtungsführung zum Orientieren des Implantats in dem ersten Knochen umfasst.

3. Vorrichtung nach Anspruch 1, die ferner ein chirurgisches Navigationssystem zum Positionieren und Ausrichten des Implantats mit Bezug auf den ersten Knochen umfasst.

4. Vorrichtung nach Anspruch 1, die ferner ein chirurgisches Navigationssystem zum Positionieren, Überwachen und Ausrichten der Einschlagvorrichtung mit Bezug auf den ersten Knochen umfasst.

5. Vorrichtung nach Anspruch 1, die ferner eine Halterung zum Anbringen an dem Anbringungsteil umfasst, wobei die Halterung zum Anbringen am zweiten Knochen ausgelegt ist.

6. Vorrichtung nach Anspruch 1, wobei das Implantat eine Acetabulumschale ist und die Einschlagvorrichtung zum Platzieren der Acetabulumschale in einem Acetabulum konfiguriert ist.

7. Vorrichtung nach Anspruch 1 oder 6, die ferner einen Adapter zum Anbringen des Implantats an der Einschlagvorrichtung umfasst.

8. Vorrichtung nach Anspruch 7, wobei der Adapter eine Drehung des Implantats einschränkt.

9. Vorrichtung nach Anspruch 6, wobei der Anbringungsteil zum direkten Anbringen am zweiten Knochen ausgelegt ist.

10. Vorrichtung nach Anspruch 6, wobei der Anbringungsteil zum indirekten Anbringen am zweiten Knochen ausgelegt ist.

11. Vorrichtung nach Anspruch 1, wobei die Einschlagvorrichtung ein pneumatischer Einschlaghammer ist.

12. Vorrichtung nach Anspruch 1, wobei die Einschlagvorrichtung ein linearer Aktuator ist.

13. Vorrichtung nach Anspruch 1, wobei die Einschlagvorrichtung ein Hydraulikkolben ist.

14. Vorrichtung nach Anspruch 1, wobei die Einschlagvorrichtung federaktiviert ist.

15. Vorrichtung nach Anspruch 1, wobei die Einschlagvorrichtung zur Aktivierung für einen einzigen Einschlagzyklus für jede Aktivierung konfiguriert ist.

16. Vorrichtung nach Anspruch 1, wobei die Einschlagvorrichtung zur Aktivierung für mehrere Einschlagzyklen konfiguriert ist, während sie aktiviert ist.

17. Vorrichtung nach Anspruch 1 bis 16, wobei der erste Knochen ein Acetabulum ist und der zweite Knochen ein Femur ist.

## Revendications

1. Appareil pour communiquer une force en vue d'asseoir un implant (252) dans un premier os (232), l'appareil comprenant :
un dispositif d'impaction (260) comportant une partie d'attachement (216) pour attacher le dispositif d'impaction à un second os (230) adjacent, le dispositif d'impaction étant structuré pour asseoir l'implant dans le premier os en conférant une force à l'implant, ladite force étant transmise par ledit implant audit premier os, **caractérisé en ce que**
la réaction à ladite force est transmise par ladite partie d'attachement audit second os.

2. Appareil selon la revendication 1, comprenant en outre un guide d'alignement externe pour orienter l'implant dans le premier os.

3. Appareil selon la revendication 1, comprenant en outre un système de navigation chirurgical pour le positionnement et l'alignement de l'implant par rapport au premier os.

4. Appareil selon la revendication 1, comprenant en outre un système de navigation chirurgical pour le positionnement, la surveillance et l'alignement du dispositif d'impaction par rapport au premier os.

5. Appareil selon la revendication 1, comprenant en outre une monture pour un attachement à la partie d'attachement, le support étant adapté pour un attachement au second os.

6. Appareil selon la revendication 1, dans lequel l'implant est une coquille acétabulaire et le dispositif d'impaction est configuré pour asseoir la coquille acétabulaire dans un acétabulum.

7. Appareil selon la revendication 1 ou 6, comprenant en outre un adaptateur pour attacher l'implant au dispositif d'impaction.

8. Appareil selon la revendication 7, dans lequel l'adaptateur limite la rotation de l'implant.

9. Appareil selon la revendication 6, dans lequel la partie d'attachement est adaptée pour un attachement direct au second os.

10. Appareil selon la revendication 6, dans lequel la partie d'attachement est adaptée pour un attachement indirect au second os.

11. Appareil selon la revendication 1, dans lequel le dispositif d'impaction est un marteau d'impaction pneumatique.

12. Appareil selon la revendication 1, dans lequel le dispositif d'impaction est un actionneur linéaire.

13. Appareil selon la revendication 1, dans lequel le dispositif d'impaction est un piston hydraulique.

14. Appareil selon la revendication 1, dans lequel le dispositif d'impaction est activé par ressort.

15. Appareil selon la revendication 1, dans lequel le dispositif d'impaction est configuré pour être activé pour un seul cycle d'impaction pour chaque activation.

16. Appareil selon la revendication 1, dans lequel le dispositif d'impaction est configuré pour être activé pour de multiples cycles d'impaction pendant qu'il est activé.

17. Appareil selon les revendications 1 à 16, dans lequel le premier os est un acétabulum et le second os est un fémur.
